(19) **Europäisches Patentamt / European Patent Office / Office européen des brevets**

(11) **EP 2 916 788 B1**

(12) **EUROPEAN PATENT SPECIFICATION**

(45) Date of publication and mention
of the grant of the patent:
**31.08.2016 Bulletin 2016/35**

(51) Int Cl.:
*A61F 13/511* (2006.01)     *A61F 13/514* (2006.01)
*B32B 5/26* (2006.01)      *D04H 1/4291* (2012.01)
*D04H 1/54* (2012.01)      *D04H 3/007* (2012.01)
*D04H 3/14* (2012.01)      *D04H 3/16* (2006.01)
*D04H 13/00* (2006.01)

(21) Application number: **13792161.5**

(22) Date of filing: **01.11.2013**

(86) International application number:
**PCT/US2013/068050**

(87) International publication number:
**WO 2014/074411 (15.05.2014 Gazette 2014/20)**

(54) **ARTICLE(S) WITH SOFT NONWOVEN WEB**

GEGENSTAND MIT WEICHEM VLIESSTOFF

ARTICLE(S) COMPRENANT UN NON-TISSÉ DOUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2012 US 201261723098 P**

(43) Date of publication of application:
**16.09.2015 Bulletin 2015/38**

(73) Proprietor: **The Procter & Gamble Company**
**Cincinnati, OH 45202 (US)**

(72) Inventors:
• **XU, Han**
  **Cincinnati, Ohio 45202 (US)**
• **FERRER, John**
  **Cincinnati, Ohio 45202 (US)**
• **DEBEER, Antonius, Lambertus**
  **Cincinnati, Ohio 45202 (US)**
• **MECL, Zdenek**
  **699 04 Anojmo (CZ)**
• **KLASKA, Frantisek**
  **699 04 Anojmo (CZ)**
• **KUMMER, Jiri**
  **699 04 Anojmo (CZ)**
• **KASPARKOVA, Pavlina**
  **699 04 Anojmo (CZ)**
• **KOHUT, Jaroslav**
  **699 04 Anojmo (CZ)**

(74) Representative: **Kremer, Véronique Marie Joséphine et al**
**Procter & Gamble Service GmbH**
**IP Department**
**Frankfurter Strasse 145**
**61476 Kronberg im Taunus (DE)**

(56) References cited:
EP-A1- 1 275 763     EP-A1- 2 034 057
WO-A1-2010/039579    WO-A1-2013/095804
WO-A2-2007/140163    US-A1- 2004 005 457

**Description**

FIELD OF THE INVENTION

The disclosure generally relates to products and other articles of manufacture that include a nonwoven web having good tactile and mechanical properties.

BACKGROUND OF THE INVENTION

The use of nonwoven webs in various products is well-known in the art. These nonwoven webs are particularly useful when used to make at least one of the numerous elements that ultimately form the product. Many of the nonwoven webs that are used in consumer products are made of various polymers such as for example polyolefins. Among other benefits, nonwoven webs made of polyolefins can enhance the tactile properties of a product such that a user or consumer perceives the product as being soft. Polymers used for the production of nonwoven textiles have characteristic properties. Nonwoven webs having fibers made of certain blends of polyolefins, such as for example a blend of polypropylene with an propylene copolymer and a softness enhancer additive are known to "feel" noticeably softer than nonwoven webs having fibers made of a single polypropylene. These softer nonwoven webs are typically made via a continuous fiber laying process such as for example carded, airlaid, or spunbond process. The nonwoven web can eventually be wound up to form a roll of the nonwoven web. The roll of nonwoven web can then be transported to another site, which can be the product manufacturing site, where the nonwoven web is unwound to make at least one element of the final product. The nonwoven web is subjected to relatively high tension along the machine direction of the web in order for the web to be unwound and further transported along the manufacturing line. This tension in the machine direction is known to cause what is referred to as "necking" of the web. Necking results in a reduction of the length of the web measured in the cross direction of the web (i.e. the direction perpendicular to the machine direction). Although "necking" can advantageously be used in some application, it can also have some adverse effect on cost and processability of the material. In particular it is observed that a nonwoven web having fibers made of certain blends of polyolefins, such as for example a blend of polypropylene with a propylene copolymer and a softness enhancer additive are prone to an unacceptable amount of necking.

It is therefore an object of the invention to provide a product that includes a nonwoven web having good tactile properties such as perceived softness and resulting in a lesser amount of necking.

It is believed that the object of the invention can be accomplished by incorporating in a product a nonwoven web having at least two fibrous layers joined to each other by bonds with a first layer including fibers made of a first composition comprising a blend of polypropylene with an propylene copolymer and a softness enhancer additive and at least a second layer including fibers made of a second composition and such that the second layer has different mechanical properties than the first layer.

WO2007140163 relates to nonwoven webs or fabrics. The nonwoven materials comprise fibers made from of a polymer blend of isotactic polypropylene and reactor grade propylene based elastomers or plastomers together with from 100 to 2500 ppm (by weight of the fiber) of a slip agent.

EP1275763 relates to Polyolefin resin-based non-woven fabric characterized by containing a fatty acid amide compound, and by having a static friction coefficient of 0.1 to 0.4

US20040005457 provides a method for producing softer fibers and nonwoven webs that includes forming a mixture comprising (i) a thermoplastic and (ii) an additive selected from the group consisting of polyethylene waxes, glyceryl monostearate, sorbitan tristearate, CATALLOY KS357 MONTELL polyolefin resin, an amide having the chemical structure $CH_3(CH_2)_7CH(CH_2)_xCONH_2$ where x is selected from 5-15 and mixtures thereof; forming the mixture into fibers and optionally creating a nonwoven web. >

SUMMARY OF THE INVENTION

One aspect of the invention is directed to an article comprising a liquid pervious layer, a liquid impervious layer, an absorbent core disposed between said liquid pervious layer and said liquid impervious layer. The article also includes a nonwoven web comprising at least a first layer of fibers that are made of a first composition comprising a first polyolefin, a second polyolefin, and a softness enhancer additive. The second polyolefin is a propylene copolymer and the said second polyolefin is a different polyolefin than the first polyolefin. The nonwoven web also includes at least a second layer of fibers that are made of a second composition. The Flexular Modulus of the second composition is greater than the Flexular Modulus of the first composition

BRIEF DESCRIPTION OF THE DRAWINGS

Fig. 1 is a schematic cross-sectional view of a nonwoven web in accordance with an embodiment of the invention;
Fig. 2 is a schematic cross-sectional view of a nonwoven web in accordance with another embodiment of the invention;
Fig. 3 is a schematic view of a process used to make one embodiment of the nonwoven web of the invention;
Figs. 4A-4C are schematic representation of bond patterns that may be applied to the nonwoven web of the invention;
Figs. 5A and 5B are enlarged picture of two diapers that include an outer cover made of two different material according to the invention; and
Fig. 6 is a schematic cross-sectional view of a product that includes one embodiment of a nonwoven web in accordance with an embodiment of the invention.

DETAILED DESCRIPTION OF THE INVENTION

As used herein, the terms "elongatable material" "extensible material" or "stretchable material" are used interchangeably and refer to a material that, upon application of a biasing force, can stretch to an elongated length of at least 150% of its relaxed, original length (i.e. can stretch to 50% more than its original length), without complete rupture or breakage as measured by Tensile Test described in greater detail below. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric." For example, an extensible but non-elastic material that has an initial length of 100mm can extend at least to 150mm, and upon removal of the force retracts to a length of at least 145mm (i.e., exhibiting 10% recovery).

As used herein, the term "film" refers generally to a relatively nonporous material made by a process that includes extrusion of, e.g., a polymeric material through a relatively narrow slot of a die. The film may be impervious to a liquid and pervious to an air vapor, but need not necessarily be so. Suitable examples of film materials are described in more detail herinbelow.

As used herein, the term "layer" refers to a sub-component or element of a web. A "layer" may be in the form of a plurality of fibers made from a single beam or a single fiber laydown step on a multibeam nonwoven machine (for example a spunbond/meltblown/spunbond nonwoven web includes at least one layer of spunbond fibers, at least one layer of meltblown fibers and at least one layer of spunbond fibers) or in the form of a film extruded or blown from a single die. The composition of a layer can be determined either by knowing the individual components of the resin composition used to form the layer, or by analyzing the composition used to make the fibers of the layer, such as via DSC or NMR.

As used herein, the term "machine direction" or "MD" is the direction that is substantially parallel to the direction of travel of a web as it is made. Directions within 45 degrees of the MD are considered to be machine directional. The "cross direction" or "CD" is the direction substantially perpendicular to the MD and in the plane generally defined by the web. Directions within 45 degrees of the CD are considered to be cross directional.

As used herein, the term "meltblown fibers" refers to fibers made via a process whereby a molten material (typically a polymer), is extruded under pressure through orifices in a spinneret or die. High velocity hot air impinges upon and entrains the filaments as they exit the die to form filaments that are elongated and reduced in diameter and are fractured so that fibers of generally variable but mostly finite lengths are produced. This differs from a spunbond process whereby the continuity of the filaments is preserved along their length. An exemplary meltblown process may be found in U.S. Pat. No. 3,849,241 to Buntin et al.

As used herein, the term "nonwoven" means a porous, fibrous material made from continuous (long) filaments (fibers) and/or discontinuous (short) filaments (fibers) by processes such as, for example, spunbonding, meltblowing, carding, film fibrillation, melt-film fibrillation, airlaying, dry-laying, wetlaying with staple fibers, and combinations of these processes as known in the art. Nonwoven webs do not have pattern formed by waving or knitting. As used herein, the term "spunbond fibers" refers to fibers made via a process involving extruding a molten thermoplastic material as filaments from a plurality of fine, typically circular, capillaries of a spinneret, with the filaments then being attenuated by applying a draw tension and drawn mechanically or pneumatically (e.g., mechanically wrapping the filaments around a draw roll or entraining the filaments in an air stream). The filaments may be quenched by an air stream prior to or while being drawn. The continuity of the filaments is typically preserved in a spunbond process. The filaments may be deposited on a collecting surface to form a web of randomly arranged substantially continuous filaments, which can thereafter be bonded together to form a coherent nonwoven fabric. Exemplary spunbond process and/or webs formed thereby may be found in U.S. Pat. Nos. 3,338,992; 3,692,613, 3,802,817; 4,405,297 and 5,665,300.

As used herein, the term "web" refers to an element that includes at least a fibrous layer or at least a film layer and has enough integrity to be rolled, shipped and subsequently processed (for example a roll of a web may be unrolled, pulled, taught, folded and/or cut during the manufacturing process of an article having an element that includes a piece of the web). Multiple layers may be bonded together to form a web.

While not intending to limit the utility of the nonwoven web described herein, it is believed that a brief description of its characteristics as they may relate to the nonwoven web manufacturing, intended use and further processing to manufacture products will help elucidate the invention. In heretofore nonwoven webs suitable for use, for example, as an element of a product such as an absorbent article as a non-limiting example, the nonwoven web typically includes fibers that are made of a polyolefin resin. Many of the products that include such nonwoven webs are at one point or another in contact with the skin of a person who may be either the user of the product or a caregiver. The use of nonwoven webs having good tactile properties has been sought after by the industry for quite some time and many such materials are known that improve the perceived softness of a product. One example of such a soft material includes a nonwoven web that is manufactured by PEGAS NONWOVENS s.r.o. under trade name PEGATEX Softblend.

This nonwoven web includes three layers of spunbond fibers that are made of a composition comprising a blend of polypropylene with an propylene copolymer and a softness enhancer additive. This nonwoven web also includes a plurality of calendering bonds, which join the layers to each other and provide the web with enough physical integrity to be processed. Although this material has good tactile properties, the resin blend used to make the fibers is relatively expensive. In addition, and as discussed further below, it is observed that this material is prone to "neck" noticeably more than other more "traditional" materials. Although necking may be desired in some applications, necking can also result in additional cost since more material end up being needed to compensate for the material length reduction along its cross direction. Since manufacturers of various products and in particular absorbent articles are under continuous pressure to reduce manufacturing cost and minimize manufacturing waste, it is believed that the nonwoven web disclosed hereinafter may be a suitable alternative to already existing nonwoven webs. The foregoing considerations are addressed by the invention, as will be clear from the detailed disclosures which follow.

It is believed that the necking of a nonwoven material is at least in part related to the Flexural Strength or Flexural Modulus of the resin composition used to make the fibers forming the nonwoven material. The flexural strength of a material is defined as its ability to resist deformation under load. Flexural modulus is a measure of stiffness or rigidity and is calculated by dividing the change in stress by the change in strain at the beginning of the test. For materials that deform significantly but do not break, the load at yield, typically measured at 5% deformation/strain of the outer surface, is reported as the Flexural Strength or flexural yield strength. The test beam is under compressive stress at the concave surface and tensile stress at the convex surface. The methodology is described for example at standard method ASTM D790. The test is stopped when the specimen reaches 5% deflection or the specimen breaks before 5%. This test also gives the procedure to measure a material's flexural modulus (the ratio of stress to strain in flexural deformation). The table 1 below lists average flexural strengths and flexural moduli values for a few examples of polymers.

Table 1

| Polymer type | Flexural Strenght (MPa) | Flexural Modulus (GPa) |
|---|---|---|
| Nylon 6 | 85 | 2,3 |
| Polyamide-Imide | 175 | 5 |
| Medium density Polyethylene | 40 | 0,7 |
| Polyethylene terephtalate (PET) | 80 | 1 |
| Polypropylene | 40 | 1,5 |
| Polystyrene | 70 | 2,5 |

These values are a measure of stiffness. Flexible materials such as elastomers or elongatable materials (typically propylene copolymers) have lower values than standard polymers (such as homopolymers). There are various methods how to affect the Flexural Modulus of a particular resin. Such methods include the addition of a filler (such as $TiO_2$), the blending of resins having different properties, and the use of various additives that are known in the art. Reference will now be made in detail to the present preferred embodiments of the invention. It should be noted that the following written description may be better understood when considered with the accompanying drawings wherein like numerals indicate the same elements throughout the views and wherein reference numerals having the same last two digits (e.g., 20 and 120) connote similar elements.

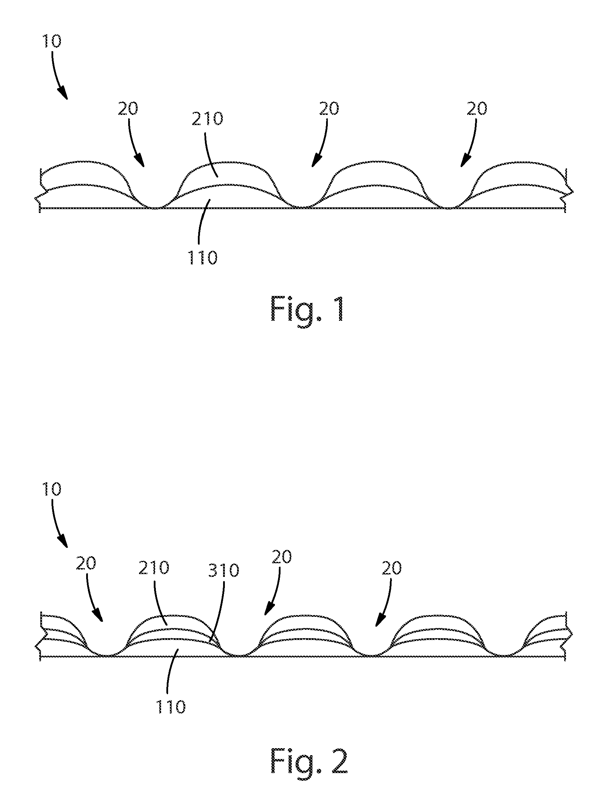

A cross-sectional view of one embodiment of the invention is schematically represented in Fig. 1 and shows a nonwoven web 10 that comprises a bottom fibrous layer 110 and a top fibrous layer 210 that is laid on top of the bottom fibrous layer 110 during the manufacturing process of the nonwoven web 10. The top and bottom fibrous layers are joined to each other at a plurality of bond sites 20, which consolidate the nonwoven web 10 and can be obtained via any well known calendering process. The bond sites 20 (or calender bonds) may have any suitable size and shape and may be formed as a repeating pattern. Non-limiting examples of suitable calender bonds and repeating patterns are disclosed *in* co-pending U.S. patent application having Serial No. U.S. 13/428,404 to Xu et al., filed on March 23, 2012, and assigned to The Procter & Gamble Company. As previously discussed, nonwoven webs having multiple fibrous layers that all include fibers having the same composition are known. Such a nonwoven web is available from PEGAS NONWOVENS s.r.o. and includes three layers of spunbond fibers where the fibers of each of the layers are made of the same composition and includes a blend of a polypropylene, a propylene copolymer and a softness enhancer additive. This particular composition will be described later in greater details. Although this nonwoven web has good tactile properties, which lead the consumer to perceive a product incorporating this web as being soft, the material is prone to necking as previously discussed. It is observed that the amount of necking can be noticeably reduced by replacing at least one of the individual fibrous layers of the nonwoven web with a fibrous layer having fibers made of a different composition than the other layer(s). In one embodiment, the top fibrous layer 210 includes fibers that are made of a first composition comprising a blend of a first polyolefin, a second polyolefin that is different than the first polyolefin and comprises a propylene copolymer, and a softness enhancer additive and the bottom fibrous layer 110 includes fibers that are made of a second composition that is different from the first composition. In one embodiment, the first polyolefin of the first composition may be a polyethylene or a polypropylene and is advantageously a polypropylene homopolymer.

It is found that a second polyolefin comprising a propylene copolymer can provide advantageous properties to the resulting nonwoven. A "propylene copolymer" includes at least two different types of monomer units, one of which is propylene. Suitable examples of monomer units include ethylene and higher alpha-olefins ranging from $C_4$-$C_{20}$, such as, for example, 1-butene, 4-methyl-1-pentene, 1-hexene or 1-octene and 1-decene, or mixtures thereof, for example. Preferably, ethylene is copolymerized with propylene, so that the propylene copolymer includes propylene units (units on the polymer chain derived from propylene monomers) and ethylene units (units on the polymer chain derived from ethylene monomers).

Typically the units, or comonomers, derived from at least one of ethylene or a C4-10 alpha-olefin may be present in an amount of 1% to 35%, or 5% to about 35%, or 7% to 32%, or 8 to about 25%, or 8% to 20%, or even 8% to 18% by weight of the propylene-alpha-olefin copolymer. The comonomer content may be adjusted so that the propylene-alpha-olefin copolymer has preferably a heat of fusion ("DSC") of 75 J/g or less, melting point of 100 °C or less, and crystallinity of 2% to about 65% of isotactic polypropylene, and preferably a melt flow rate (MFR) of 0.5 to 90 dg/min.

In one embodiment, the propylene-alpha-olefin copolymer comprises of ethylene-derived units. The propylene-alpha-olefin copolymer may contain 5% to 35%, or 5% to 20%, or 10% to 12%, or 15% to 20%, of ethylene-derived units by weight of the propylene-alpha-olefin copolymer. In some embodiments, the propylene-alpha-olefin copolymer consists essentially of units derived from propylene and ethylene, i.e., the propylene-alpha-olefin copolymer does not contain any other comonomer in an amount typically present as impurities in the ethylene and/or propylene feedstreams used during polymerization or an amount that would materially affect the heat of fusion, melting point, crystallinity, or melt flow rate of the propylene-alpha-olefin copolymer, or any other comonomer intentionally added to the polymerization process.

The propylene-alpha-olefin copolymer may have a triad tacticity of three propylene units, as measured by 13C NMR, of at least 75%, at least 80%, at least 82%, at least 85%, or at least 90%. The "Triad tacticity" is determined as follows. The tacticity index, expressed herein as "m/r", is determined by 13C nuclear magnetic resonance ("NMR"). The tacticity index m/r is calculated as defined by H. N. Cheng in 17 MACROMOLECULES 1950 (1984), incorporated herein by reference. The designation "m" or "r" describes the stereochemistry of pairs of contiguous propylene groups, with "m" referring to meso and "r" referring to racemic. An m/r ratio of 1.0 generally describes a syndiotactic polymer, and an m/r ratio of 2.0 generally describes an atactic material. An isotactic material theoretically may have a m/r ratio approaching infinity, and many by-product atactic polymer have sufficient isotactic content to result in an m/r ratio of greater than 50.

The propylene-alpha-olefin copolymer may have a heat of fusion ("Hf"), as determined by Differential Scanning calorimetry ("DSC"), of 75 J/g or less, 70 J/g or less, 50 J/g or less, or even 35 J/g or less. The propylene-alpha-olefin copolymer may have a Hf of at least 0.5 J/g, 1 J/g, or at least 5 J/g. The "DSC" is determined as follows. About 0.5 grams of polymer is weighed and pressed to a thickness of about 15 to 20 mils (about 381-508 microns) at about 140-150°C, using a "DSC mold" and MYLAR(TM) film as a backing sheet. The pressed polymer sample is allowed to cool to ambient temperatures by hanging in air (the MYLAR(TM) film backing sheet is not removed). The pressed polymer sample is then annealed at room temperature (about 23-25°C.) for 8 days. At the end of this period, a 15-20 mg disc is removed from the pressed polymer sample using a punch die and is placed in a 10 microliter aluminum sample pan. The disc sample is then placed in a DSC (Perkin Elmer Pyris 1 Thermal Analysis System) and is cooled to -100°C. The sample is heated at about 10°C/min to attain a final temperature of 165°C. The thermal output, recorded as the area under the melting peak of the disc sample, is a measure of the heat of fusion and can be expressed in Joules per gram (J/g) of polymer and is automatically calculated by the Perkin Elmer system. Under these conditions, the melting profile shows two maxima, the maximum at the highest temperature is taken as the melting point within the range of melting of the disc sample relative to a baseline measurement for the increasing heat capacity of the polymer as a function of temperature.

The propylene-alpha-olefin copolymer may have a single peak melting transition as determined by DSC. In one embodiment, the copolymer has a primary peak transition of 90° C. or less, with a broad end-of-melt transition of about 110° C. or greater. The peak "melting point" ("Tm") is defined as the temperature of the greatest heat absorption within the melting range of the sample. However, the copolymer may show secondary melting peaks adjacent to the principal peak, and/or at the end-of-melt transition. For the purposes of this disclosure, such secondary melting peaks are considered together as a single melting point, with the highest of these peaks being considered the Tm of the propylene-alpha-olefin copolymer. The propylene-alpha-olefin copolymer may have a Tm of 100 °C or less, 90 °C or less, 80 °C or less, or 70 °C or less. The propylene-alpha-olefin copolymer may have a density of 0.850 to 0.920 g/cm3, 0.860 to 0.900 g/cm3, or 0.860 to 0.890 g/cm3, at room temperature as measured per ASTM D-1505.

The propylene-alpha-olefin copolymer may have a melt flow rate ("MFR"), as measured according to ASTM D1238, 2.16 kg at 230° C., of at least 0.2 dg/min. In one embodiment, the propylene-alpha-olefin copolymer MFR is 0.5 to 5000 dg/min, about 1 to 2500 dg/min, about 1.5 to 1500 dg/min, 2 to 1000 dg/min, 5 to 500 dg/min, 10 to 250 dg/min, 10 to 100 dg/min, 2 to 40 dg/min, or 2 to 30 dg/min.

The propylene-alpha-olefin copolymer may have an Elongation at Break of less than 2000%, less than 1000%, or less than 800%, as measured per ASTM D412.

The propylene-alpha-olefin copolymer may have a weight average molecular weight (Mw) of 5,000 to 5,000,000 g/mole, preferably 10,000 to 1,000,000 g/mole, and more preferably 50,000 to 400,000 g/mole; a number average molecular weight (Mn) of 2,500 to 2,500,00 g/mole, preferably 10,000 to 250,000 g/mole, and more preferably 25,000 to 200,000 g/mole; and/or a z-average molecular weight (Mz) of 10,000 to 7,000,000 g/mole, preferably 80,000 to 700,000 g/mole, and more preferably 100,000 to 500,000 g/mole. The propylene-alpha-olefin copolymer may have a molecular weight distribution ("MWD") of 1.5 to 20, or 1.5 to 15, preferably 1.5 to 5, and more preferably 1.8 to 5, and most preferably 1.8 to 3 or 4. The "Molecular weight (Mn, Mw, and Mz)" and "MWD" can be determined as follows and as described in Verstate et al., 21 MACROMOLECULES 3360 (1988). Conditions described herein govern over published test conditions. Molecular weight and MWD are measured using a Waters 150 gel permeation chromatograph equipped with a Chromatix KMX-6 on-line light scattering photometer. The system is used at 135°C. with 1,2,4-trichlorobenze as the mobile phase. Showdex (Showa-Denko America, Inc.) polystyrene gel columns 802, 803, 804, and 805 are used. This technique is discussed in Verstate et al., 21 MACROMOLECULES 3360 (1988). No corrections for column spreading are employed; however, data on generally acceptable standards, e.g., National Bureau of Standards Polyethylene 1484, and anionically produced hydrogenated polyisoprenes (an alternating ethylenepropylene copolymer) demonstrate that such corrections on Mw/Mn or Mz/Mw are less than 0.05 units. Mw/Mn was calculated from an elution time-molecular relationship whereas Mz/Mw was evaluated using the light scattering photometer. The numerical analysis can be performed using the commercially available computer software GPC2, MOLWT2 available from LDC/Milton Roy-Rivera Beach, Fla.Examples suitable propylene-alpha-olefin copolymers are available commercially under the trade names VISTAMAXX® (ExxonMobil Chemical Company, Houston, Tex., USA), VERSIFY® (The Dow Chemical Company, Midland, Mich., USA), certain grades of TAFMER® XM or NOTIO® (Mitsui Company, Japan), and certain grades of SOFTEL® (Basell Polyolefins of the Netherlands). The particular grade(s) of commercially available propylene-alpha-olefin copolymer suitable for use in the invention can be readily determined using methods applying the selection criteria in the above.

Propylene copolymers have a good mixability with other polyolefins and in particular with propylene homopolymers, where depending on the mutual ratio of both constituents it is possible to prepare a material exhibiting various properties. A propylene copolymer is soft to touch and the nonwoven textile produced from it has good drapeability and is easy to bend. On the other hand polypropylene provides strength and reduces the plasticity of the material. Examples of composition that are suitable for the manufacturing of fibrous nonwoven materials can include at least 60%, at least 70%, at least 75%, or at least 80% by weight of the composition of polypropylene homopolymer, and at least 10%, at least 12%, at least 14% by weight of the propylene copolymer. The described composition is generally drapable and soft but als maintains the required mechanical properties. However it is found that it can feel rough to the touch and can be described as "rubbery." In particular, propylene-alpha-olefin copolymers, particularly propylene-ethylene copolymers, can be tackier than conventional fibers made from polyolefins such as polyethylene and polypropylene.

It is found that the addition of a softness enhancer additive can be advantageous to reduce the tacky or rubbery feel of fibers that are made of a composition that includes a blend of the first and second polyolefin previously described. The softness enhancer additive may be added to the composition in neat form, diluted, and/or as a masterbatch in, for example, polyolefin polymers such as polypropylene, polystyrene, low density polyethylene, high density polyethylene, or propylene-alpha-olefin copolymers.

A first composition suitable to make fibers as described herein may also contain one or more softness enhancer additive, which can be present in an amount of between 0.01% to 10%, or between 0.03% to 5%, or even between 0.05% to 1% by weight of the fibers. Once the fiber are spun to form a nonwoven, some of the softness enhancer additive may volatilize and no longer be present in the same amount in the fibers forming the nonwoven, It is also believed that some of the softness enhancer additive may migrate from the interior portion of the fiber to the outer surface of the fiber. Without intending to be bound by any theory, it is believed that this migration of the additive to the outer surface of the fiber may contribute to the perception of softness that a user experiences when she touches the nonwoven material.

In one embodiment, the softness enhancer additive is an organic amine compound, i.e., contains an amine group bound to a hydrocarbon group. In another embodiment, the softness enhancer additive is a fatty acid amine or a fatty acid amide. In some embodiments, the softness enhancer additive may have one or more paraffinic or olefinic groups bound to a nitrogen atom, forming an amine or an amide compound. The paraffinic or olefinic group may be, for example, a polar or ionic moiety as a side chain or within the amine/amide backbone. Such polar or ionic moieties can include hydroxyl groups, carboxylate groups, ether groups, ester groups, sulfonate groups, sulfite groups, nitrate groups, nitrite groups, phosphate groups, and combinations thereof.

In one embodiment, the softness enhancer additive is an alkyl-ether amine having the formula $(R'OH)_{3-x}NR_x$, wherein R is selected from the group consisting of hydrogen, C1-40 alkyl radicals, C2-40 alkylethers, C1-40 alkylcarboxylic acids, and C2-40 alkylesters; R' is selected from the group consisting of C1-40 alkyl radicals, C2-40 alkylethers, C1-40 carboxylic acids, and C2-40 alkylesters; and x is 0, 1, 2 or 3, preferably 0 or 1, more preferably 1. In one embodiment, R is selected from the group consisting of hydrogen and C5-40 alkyl radicals; and R' is selected from the group consisting of C5-40 alkyl radicals and C5-40 alkylethers.

In another embodiment, the softness enhancer additive is an amide-containing compound having the formula: $RCONH_2$, wherein R is a C5-23 alkyl or alkene. In another embodiment, the softness enhancer additive is a fatty acid amide having the formula: $(R'CO)_{3-x}NR''_x$, wherein R'' is selected from the group consisting of hydrogen, C10-60 alkyl radicals and C10-60 alkene radicals and substituted versions thereof; R' is selected from the group consisting of C10-60 alkyl radicals, C10-60 alkene radicals, and substituted versions thereof; and x is 0, 1, 2 or 3, preferably 1 or 2, more preferably 2. As used herein, an "alkene" radical is a radical having one or more unsaturated double-bonds in the radical chain (e.g., $--CH_2CH_2CH_2CH_2CH=CHCH_2CH_2CH_2CH._{sub-}2CH_2CH_3$), and "substituted" means substitution anywhere along the hydrocarbon chain of a hydroxyl group, carboxyl group, halide, or sulfate group.

In some embodiments, the softness enhancer additive contains an unsaturated amide. In one embodiment, the unsaturated amide-containing softness enhancer additive has the formula: $RCONH_2$, wherein R is a C5-23 alkene. In another embodiment, the unsaturated amide-containing softness enhancer additive has the formula: $(R'CO)_{3-x}NR''_x$, wherein R'' is selected from the group consisting of hydrogen, C10-60 alkyl radicals and C10-60 alkene radicals and substituted versions thereof; R' is selected from the group consisting of C10-60 alkene radicals and substituted versions thereof; and x is 0, 1, 2 or 3, preferably 1 or 2, more preferably 2. In some embodiments, the unsaturated amide-containing softness enhancer additive is at least one of palmitoleamide, oleamide, linoleamide, or erucamide. In other embodiments, the unsaturated amide-containing softness enhancer additive is at least one of oleamide or erucamide. In the preferred embodiment the softness enhancer additive contains erucamide.

Non-limiting examples of softness enhancer additives include bis(2-hydroxyethyl) isodecyloxypropylamine, poly(5)oxyethylene isodecyloxypropylamine, bis(2-hydroxyethyl) isotridecyloxypropylamine, poly(5)oxyethylene isotridecyloxypropylamine, bis(2-hydroxyethyl) linear alkyloxypropylamine, bis(2-hydroxyethyl) soya amine, poly(15)oxyethylene soya amine, bis(2-hydroxyethyl) octadecylamine, poly(5)oxyethylene octadecylamine, poly(8)oxyethylene octadecylamine, poly(10)oxyethylene octadecylamine, poly(15)oxyethylene octadecylamine, bis(2-hydroxyethyl) octadecyloxypropylamine, bis(2-hydroxyethyl) tallow amine, poly(5)oxyethylene tallow amine, poly(15)oxyethylene tallow amine, poly(3)oxyethylene-1,3-diaminopropane, bis(2-hydroxyethyl) cocoamine, bis(2-hydroxyethyl)isodecyloxypropylamine, poly(5)oxyethylene isodecyloxypropylamine, bis(2-hydroxyethyl) isotridecyloxypropylamine, poly(5)oxyethylene isotridecyloxypropylamine, bis(2-hydroxyethyl) linear alkyloxypropylamine, bis(2-hydroxyethyl) soya amine, poly(15)oxyethylene soya amine, bis(2-hydroxyethyl) octadecylamine, poly(5)oxyethylene octadecylamine, poly(8)oxyethylene octadecylamine, poly(10)oxyethylene octadecylamine, poly(15)oxyethylene octadecylamine, bis(2-hydroxyethyl) octadecyloxypropylamine, bis(2-hydroxyethyl) tallow amine, poly(5)oxyethylene tallow amine, poly(15)oxyethylene tallow amine, poly(3) oxyethylene-1,3-diaminopropane, bis(2-hydroxethyl) cocoamine, valeramide, caproicamide, erucamide, caprylicamide, pelargonicamide, capricamide, lauricamide, lauramide, myristicamide, myristamide, palmiticamide, palmitoleamide, palmitamide, margaric (daturic) amide, stearicamide, arachidicamide, behenicamide, behenamide, lignocericamide, linoleamide, ceroticamide, carbocericamide, montanicamide, melissicamide, lacceroicamide, ceromelissic (psyllic) amide, geddicamide, 9-octadecenamide, oleamide, stearamide, tallow bis(2-hydroxyethyl)amine. cocobis(2-hydroxyethyl)amine, octadecylbis(2-hydroxyethyl)amine, oleylbis(2-hydroxyethyl)amine, ceroplastic amide, and combinations thereof.

Commercial examples of useful softness enhancer additives include ATMER® compounds (Ciba Specialty Chemicals), ARMID®, ARMOFILM® and ARMOSLIP® compounds and NOURYMIX concentrates (Akzo Nobel Chemicals), CROTAMID® compounds (Croda Universal Inc), CESA SLIP® compounds (Clariant). Further examples of softness enhancer additives include compounds from A.Schulman, Germany, Techmer, USA, or Ampacet, USA.

Compositions useful in the invention may include one or more different softness enhancer additives. For example, in one embodiment a composition may contain one or more unsaturated amide-containing softness enhancer additives, and in another embodiment one or more unsaturated amide-containing softness enhancer additives and one or more saturated amide-containing softness enhancer additives. In some embodiments, a composition includes a combination of low molecular weight (Mw) and thus faster migrating amides, e.g., erucamide or oleamide, and higher molecular weight (Mw) and thus slower migrating amides, e.g., behenamide or stearamide. It should be noted, that compounds that are suitable as softness enhancer additives, such as for example amide additives, may sublimate (i.e. transform directly from a solid state to a gaseous state) when subjected to high temperatures. One skilled in the art will appreciate that the sublimation level may depend on the additive temperature and partial pressure of additive vapors over the surface exposed to the outside environment. One skilled in the art will also appreciate that the processing temperatures should remain lower than the TGA (i.e. Thermogravimetric analysis) Rapid weight loss temperature of the components. Surprisingly it is been found, that when softness enhancer additives of the amide type are added in a spun melting process, it is advantageous to maintain the process temperatures at a level well below the TGA Rapid weight loss temperature. In particular, it is believed that the temperature of the molten composition ahead of the spinnerets should be at least 20°C lower, or even 25°C lower than the TGA Rapid weight loss temperature of the softness enhancer additive. The TGA Rapid weight loss temperature for various substances can be found for example in "Plastics additives: an industrial guide" written by Ernest W.Flick.

Without wishing to be bound by theory it is believed that this sublimation of the additive can be caused by particular process conditions during fiber production. As in typical nonwoven manufacturing processes, the polymer composition is molten and brought to a particular temperature, which enables the composition to flow and be extruded through spinnerets in order to form fibers. The newly formed fibers are then quenched at a much lower temperature by air, which flows against the fibers' outer surface. When the molten composition is heated to a temperature, which causes the softness enhancer additive to overheat, and the additive may evaporate/sublimate from the outer surface of solidifying fiber. Because of the rapid and constant air flow, the partial pressure is kept to a relative low level, which favors evaporation/sublimation of the softness enhancer additive than one would otherwise expect from TGA values. The following table 2 provides temperatures for several amides.

Table 2

| TGA Weight Loss of Amides | | | | |
|---|---|---|---|---|
| Type | Softness Enhancer Additive | % total Weight Loss | Temperature when Weight Loss begins (°C) | Temperature when RapidWeight Loss begins (°C) |
| Primary | Oleamide | 99.3 | 195 | 250 |
| | Erucamide | 94.8 | 220 | 280 |
| Secondary | Oleylpalmitamide | 11.8 | 225 | 300 |
| Bisamide | Ethylenebisoleamide | 11.6 | 220 | 305 |

Notwithstanding the improvements provided by such additives, compositions that include the additive still exhibit certain drawbacks when compared to others such as homopolymers of polypropylene. As previously discussed, it can be desirable to minimize the amount of nekdown of a web, in particular when the web is subject to tension it its machine direction. In addition, it is observed that the webs made of a composition that includes a copolymer polypropylene with a softness enhancer additive tends to have a lower coefficient of friction. Such a lower coefficient of friction can lead to unexpected difficulties in the handling of the web, such as winding, which may become more difficult and/or require a higher winding tension. This may ultimately lead to undesired compaction of the web. Henceforth, in at least some aspects, the invention aims at providing a layered structure which at least reduces, if not entirely eliminate such drawbacks while maintaining its benefits.

In one embodiment, the second composition used to make the fibers of a second layer is chosen from a resin or in the alternative a blend of resins such that a fibrous layer made from this second composition is prone to less necking than a fibrous layer made from the first composition. A non-limiting example of a second composition that may be advantageously used to make the fibers of the second layer, and which can be the bottom layer 110 include a composition, which contains less propylene copolymer by weight of the second composition than the amount of propylene copolymer by weight of the first composition. A second composition may contain less than 10%, or less than 8%, or less than 5%, or even less than 1% by weight of the second composition of a propylene copolymer. One of ordinary skill will appreciate that it may be advantageous for second composition to only contain an insignificant amount of, or no propylene copolymer in order to form a second fibrous layer that is less prone to necking than a first fibrous layer in particular when the second fibrous layer is subjected to a force oriented substantially in the machine direction of the second fibrous layer. A second composition may contain at least 80%, or at least 90%, or even at least 97% by weight of the second composition of a polypropylene homopolymer. In addition, it can be advantageous to select the first and second compositions such that the resulting nonwoven web formed by the first and second fibrous layers is elongatable but substantially non-elastic. Such nonwoven web can be particularly advantageous when the nonwoven web is joined to another material such as a film and the resulting laminate is subjected to mechanical strain in its cross-machine direction such as ring-rolling.

As further described in greater details below, the nonwoven web 10 is subjected to calendering by being advanced through the nip formed by two calendering rolls. One of the rolls is referred to as a smooth roll and includes a smooth outer surface which is in contact with the bottom layer 110 of the nonwoven web during calendering. The other roll is referred to as an embossing roll and includes a plurality of protrusions, which engage the top fibrous layer 210 of the nonwoven web and "pinch" the top and bottom layers to form bond sites that jointhe fibrous layers forming the nonwoven web. Each of the smooth and embossing rolls is preferably heated in order to melt the fibers made of the first and second compositions at the local bond sites 20 and to form a coherent nonwoven web. The melting of the fibers results in the formation of film like structures at the bond site that are each surrounded by a "gusset" like structure. One of ordinary skill will understand that the calendering process and the resulting calendering bonds provide the nonwoven web with a first textured surface and a second surface opposite the first textured surface. This second surface of the nonwoven web (i.e. the surface of the web which was against the smooth roll during calendering) can be substantially flat as opposed to the first surface which has a more pronounced three-dimensional texture.

A nonwoven web having a bottom fibrous layer having fibers made of a first composition comprising a polypropylene, an propylene copolymer and a softness enhancer additive, and a top fibrous layer made of a second composition that is different from the first composition and prone to less necking is also part of the scope of the invention. One of ordinary skill will understand that in this configuration, the embossed roll will engage the top fibrous layer having fibers made of the second composition (i.e. the layer that is prone to less necking) whereas the bottom layer that comprises fibers made of the first composition will lay against the smooth roll during calendering. Without intending to be bound by any theory, it is believed that such an arrangement of the layers relative to the embossed and smooth rolls result in a nonwoven web that has less fuzz in comparison to a nonwoven web whose top layer with fibers made of the first composition is engaged by the embossed roll during calendering. Said differently, it can be advantageous for the bottom layer having a substantially flat surface to include fibers that are made of the first composition and are prone to necking, while the top layer having a substantially textured surface. It should be understood that no matter its location on the nonwoven web relative to the embossed and smooth roll, the fibrous layer that includes fibers made of a first composition comprising a first polyolefin, a second polyolefin that comprises a propylene copolymer and is different than the first polyolefin, and a softness enhancer additive is preferably present and forms the surface of the product or article that is intended to be contacted by a person skin. And it is also believed that a nonwoven web having a top fibrous layer 210 having fibers made of a first composition comprising a first polyolefin, a second polyolefin that comprises a propylene copolymer and is different than the first polyolefin, and a softness enhancer additive, and a bottom fibrous layer 110 made of a second composition that is different from the first composition and preferably comprises less than 10% by weight of a propylene copolymer has particularly advantageous tactile and softness properties when the fibrous layer that includes fibers made of a first composition is the layer, which contacts the embossing roll during the calendering process. Without intending to be bound by any theory, it is believed that the three-dimensional texture imparted to the nonwoven web during the calendering process further enhances a person's perception of softness of the nonwoven web. In addition, it is believed that a person's fingers or skin are less likely to come in contact with the film like structures and gussets that are present at the bond sites on a fibrous layer 110 and may feel no as soft to the touch. Figure 2 illustrate a schematic cross-section view of another embodiment of a nonwoven web 10 that includes a bottom fibrous layer 110, a top fibrous layer 210 and at least one intermediate fibrous layer 310 disposed between the top and bottom fibrous layers. As previously discussed, the nonwoven web 10 may also include a plurality of calendering bonds that join the layers to each other and provide mechanical integrity to the nonwoven web. As previously discussed, either the top and/or bottom fibrous layers can include fibers made of a first composition such as any of the first compositions previously described and may comprise a first polyolefin, a second polyolefin that comprises a propylene copolymer and is different than the first polyolefin, and a softness enhancer additive. But it may also be advantageous for the fibrous layer that contacts the embossing roll during calendering to be the layer that includes fibers made of a first composition comprising a first polyolefin, a second polyolefin that comprises a propylene copolymer and is different than the first polyolefin, and a softness enhancer additive. In applications, it may also be advantageous for the fibrous layer that contacts the smooth roll during calendering to be the layer that includes fibers made of a first composition such as any of the first compositions previously discussed. As previously discussed, it is advantageous for at least one of the top, bottom, and intermediate fibrous layers to include fibers that are made of a second composition that is different from the first composition in particular when the second composition is chosen from one of the compositions previously described. The addition of at least one intermediate layer 310 provides its own benefits such as providing the nonwoven web with greater basis weight homogeneity and/or the inclusion of a layer that influences the mechanical properties of the overall nonwoven web. Every combination or arrangement of the layers is contemplated to be within the scope of the invention as long as at least one of the fibrous layers forming the nonwoven web includes fibers made of a composition comprising a first polyolefin, a second polyolefin that comprises a propylene copolymer and is different than the first polyolefin, and a softness enhancer additive. Said differently, the fibers of each of the individual layers of the nonwoven web are not all made of the same composition. A nonwoven formed by different fibrous layers having different properties can be obtained by careful selection of the composition used to make the fibers of the individual layers. The table below includes examples of compositions that may be used for individual layers of a nonwoven web made of three layers and the expected benefit obtained depending on the composition selected. Examples of functional layering by selecting layer composition. It will be understood that the table 3 below can be used to select the layers of nonwoven materials having two, three or more layers.

Table 3

|  | Polypropylene homopolymer | Blend of polypropylene homopolymer and polypropylene copolymer | Blend of polypropylene homopolymer and polypropylene copolymer and softness enhancer additive |
|---|---|---|---|
| "user side" - Layer contacted by user or e consumer's skin | Strength Loft/caliper Higher friction Lower neckdown | Good extension properties Increased neckdown High friction surface | Softness Drape |
| Intermediate layer | Strength Loft/caliper Lower neckdown | Good extension properties Increased neckdown | Softness Drape |
| Layer facing away from user or consumer's skin | Strength Loft/caliper Higher friction with improved winding Improved gluing/joining properties Lower neckdown | Good extension properties Increased neckdown Improved gluing/joining properties | Softness Drape |

In one embodiment, an intermediate fibrous layer 310 can include fibers made of a third composition that comprises the same components as the first composition in the same or different proportions than the first composition. In another embodiment, an intermediate fibrous layer 310 can include fibers made of a third composition that is different from said first composition. In this instance, the third composition may be substantially the same as the second composition or may instead be different from both the first and second compositions.

It should be noted that there exist multiple combinations of such two-, three- or even multi-layer composites, which may - depending on the particular application may be provided their own benefit. For explanatory purposes the first composition comprising a blend of a first polyolefin, a second polyolefin and a softness enhancer additive is denoted "B" and a second composition comprising a third polyolefin is denoted "P". Any other layer without further specification is referred to as "X." Thus, for a two-layered material, there is one option only: PB - material with one side with improved softness intend to be positioned towards a user and another side to be for example laminated or glued to some other part. A three layered material offers generally three possible arrangements: BXP, BPX and XBP. Both BXP and BPX are preferred options when the nonwoven material is intended to contact the skin of a consumer because the first composition "B" is placed on outer layer, such that it is available for contact with a user's or consumer's skin while the "P" layer decreases the neck down of the final web. The "XBP" option may not be as advantageous in applications relying on the composition of the B layer for softness, because the first composition "B" is "hidden" between two other facing layers, such that it may not be available for contact with the consumer's skin. Considering repeating layers in the configuration (i.e. "X" can be specified as "B" or "P", more options arise, namely "PPB", "PBB", "BPB" and "PBP", where again first three options are preferred over the last one ("PBP") as therein the first composition is not exposed to the user.

Reducing for simplicity the benefits, the "B" layer provides a soft and pleasant touch and the "P" layer provides lower neckdown and can also provide advantages from a material processing perspective and for further web converting. When the "P" layer is hidden as for example in BPB configuration, thematerial can very suitably be used by itself to make an element such as the leg cuff material in absorbent articles, as both facing layers can be touched by users and the "P" layer in the middle still provide mechanical properties and decrease neckdown under required limit.

Considering multi-layer materials, number of options increase rapidly with number of layers. Also, all layers can be made by same method (for example spunbond fabric) and it was not considered that typically a bonded nonwoven web has a textured side and smooth side, as will be discussed in more detail.. It should be noted that each layer consist of fibers, that can be produced by different methods (e.g. essentially endless spunmelt fibres like spunbond, meltblown, advanced meltblown, BIAX meltblown fibres etc, or staple fibers well known in the art, or for example fibers from meltfibrilation etc.). Position of textured and smooth side of the nonwoven is not limited to any layer, so for example material with following compositions can be produced (not limiting to following list): (smooth) PXMMMB (textured) or (smooth) BPP (textured) or (smooth) PXB (textured) or (smooth) PXMNB(textured) or (smooth) PMNMBB (textured) or (smooth) PXMFFB (textured) where "M" stands for meltblown fibers, "F" for fibers from meltfibrilation process and "N" for nanofibers.

As exemplified in the table 4 provided below, the fibers of one of the layers may have a different denier than the denier of the fibers of at least one of the other layer(s) forming the nonwoven web. It is believed that a top fibrous layer 210 having fibers with a lower denier than the denier of the fibers of the bottom and/or intermediate fibrous layers 110 and 310 further enhances the tactile properties of the overall nonwoven web in particular when the fibrous layer with a lower denier has substantially the same basis weight as the other fibrous layer(s) with a higher denier. Without intending to be bound by any theory, it is believed that at substantially equal basis weight, a fibrous layer with a lower denier than another fibrous layer includes a greater number of fibers. And it is also believed that a greater number of fibers, that are in particular made of a first composition comprising a first polyolefin, a second polyolefin that comprises a propylene copolymer and is different than the first polyolefin, and a softness enhancer additive, enhances a person's perception of softness of a product incorporating the nonwoven web.

Depending on the intended use of the nonwoven web by itself or in a product, it may be advantageous for the web to have additional specific properties such as for example enhanced hydrophilicity, enhanced hydrophobicity, antistatic properties, so called "alcohol repellency" included non polar liquids repellency, color etc. The desired property(ies) can be obtained generally either by adding active additive(s) into the resin composition and/or by treating the fibers after the fibers are formed (for example via a wet treatment).

As was briefly described above, tactile properties such as the perception of softness by a consumer or a user can be difficult to express via single measurement. Without intending to be bound by any theory, it is believed that the Material Factor described herein below, and which is obtained by measuring four physical parameters is a good predictor of how a person will perceive the softness of a material. The four physical properties that are relied upon to determine the Material Factor for a particular nonwoven material are the material Neck Down Modulus, the material Caliper, the material Basis Weight and the material Coefficient of Friction. The Material Factor is calculated via the following equation:

$$\text{Material Factor} = \frac{10 \times \text{Neck Down Modulus} \times \text{Caliper}}{\text{Basis Weight} \times (\text{Coefficient of Friction})^4}$$

The Material Factor is expressed in $Nm^2/g$, the Neckdown Modulus is expressed in N/cm, the Caliper is expressed in mm, the Basis Weight is expressed in $g/m^2$ and the Coefficient of Friction is unit-less. It should be noted that the Coefficient of Friction used in this equation is the Static Coefficient of Friction measured along the Machine Direction of the web sample. It should be noted that the Coefficient of Friction used in the Material Factor equation is the Static Coefficient of Friction measured between two nonwovens along the Machine Direction of the samples.

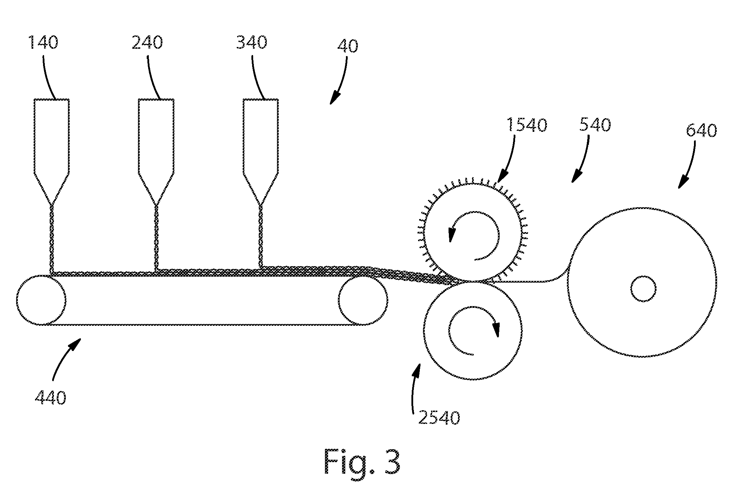

Several nonwoven webs having three fibrous layers are made and tested for different properties. Each of the nonwoven webs is made according to a spunbonding process that is schematically represented in Figure 3. The process line 40 includes a first beam 140, a second beam 240 and a third beam 340 that are each adapted to produce spunbond fibers. Each of the beams 140, 240 and 340 may be connected to at least one extruder (not shown) that feeds the desired compositions to spinnerets of the beams as it is well known in the art. It will be appreciated that various spinneret configurations may be used to obtain fibers having different cross-sectional shapes and/or diameters/denier. The spunbond fibers that are produced by the first beam 140 are deposited on a forming surface 440 which can be a foraminous belt. The forming surface 440 may be connected to a vacuum in order to draw the fibers onto the forming surface. The spunbond fibers produced by the first beam 140 form the bottom fibrous layer 110 previously described in the context of Figure 2. The spunbond fibers that are produced by the second beam 240 are deposited onto the fibers previously produced by the first beam 140. The spunbond fibers produced by the second beam 240 form the intermediate fibrous layer 310 previously described in the context of Figure 2. It will be appreciated that additional intermediate fibrous layers may be formed by simply adding additional beams such as spunbond, meltblown, advanced meltblown and melt-film-fibrillation. Any of the intermediate fibrous layers may be made of spunbond fibers. But other fibers, such as for example meltblown and/or sub-micron fibers may be included as an intermediate layer. The spunbond fibers that are produced by the third beam 340 are deposited onto the fibers previously produced by the second beam 240. The spunbond fibers produced by the third beam 340 form the top fibrous layer 210 previously described in the context of Figure 2. After each of the fibrous layers of the nonwoven web is formed, the web is then transported to a calendering station 540. The calendering station 540 includes a first and second rotating (or calendering) rolls 1540, 2540 such that at least one of the first, and second rolls includes a plurality of protrusions (shown in roll 1540) that form the bond sites 20 that are preferably organized in a repeating pattern. It can be advantageous for the second roll 2540 to have a substantially smooth surface in order to impart a well defined pattern onto the nonwoven web. The first and second rotating rolls may be heated, preferably to a temperature that is greater than the melt temperature of each of the compositions used to make the fibers of the fibrous layers. After the calendering process the nonwoven web can be subjected to further treatment (e.g. wet treatment and drying). The nonwoven web is then moved to a storage station 640 where the web rolled such that it can be conveniently transported to a storage site or an article manufacturing site.

It will also be appreciated that the final nonwoven properties can be adjusted by changing the manufacturing line settings. For example calendering that is conducted at a too high temperature can result in a nonwoven material having inferior tactile properties. But calendering that is conducted at a temperature that is too low can result in a nonwoven web having inferior tensile properties and which is prone to neck down. Several samples of nonwoven webs are made in accordance with the process described in Figure 3 and tested for different properties. The results of these tests are summarized in Table 4 below. In this table, a composition that includes a blend of first polyolefin, a second polyolefin and a softness enhancer additive is denoted as "B" and another composition comprising a third polyolefin is denoted as "P" and "V" refers to a blend of a first and a second polyolefin, which does not include a softness enhancer additive. The abbreviation "+" denotes an increased amount of copolymer. It should also be noted that the first layer identified in the samples is the layer that contacts the smooth roll during the calendering of the web and third layer is the layer that contacts the embossed roll during the calendering process.

Table 4

| Material Sample | Composition code | Basis Weight (g/m2) | Fuzz (mg/cm2) | Thickness / Caliper (mm) | COF MD stat (-) | Neckdown modulus (-) | Material Factor |
|---|---|---|---|---|---|---|---|
| 1 | B B B | 24.9 | 0.21 | 0.35 | 0.38 | 2.62 | 1.75 |
| 2 | B P B | 25.5 | 0.14 | 0.38 | 0.35 | 3.53 | 3.60 |
| 3 | P B+ B+ | 24.8 | 0.18 | 0.38 | 0.36 | 4.60 | 4.17 |
| 4 | P V B | 25.8 | 0.16 | 0.38 | 0.34 | 4.50 | 5.17 |
| 5 | P V B | 25.1 | 0.21 | 0.38 | 0.34 | 5.88 | 6.71 |
| 6 | P B B | 24.8 | 0.16 | 0.32 | 0.37 | 6.75 | 4.55 |
| 7 | P P B | 24.7 | 0.10 | 0.35 | 0.41 | 8.29 | 4.23 |
| 8 | B+ P P | 24.7 | 0.13 | 0.34 | 0.45 | 8.10 | 2.75 |
| 9 | P P P | 25.4 | 0.16 | 0.43 | 0.55 | 9.89 | 1.82 |

Sample 1 - BBB

A spunbond type nonwoven web is produced via a continuous process using three beams. Each of the beams is fed a composition that consists essentially of about 82% by weight of a polypropylene homopolymer (Tatren HT2511 from Slovnaft Petrochemicals), 16% by weight of propylene copolymere (Vistamaxx 6202 from Exxon) and about 2% by weight of a softness enhancer additive containing 10% erucamide (CESA PPA0050079 from Clariant). For all three beams the temperature of polymeric composition measured after the extruder zone is between 245-252°C. Melt spun monocomponent filaments with a fiber diameter of between 15-25 $\mu$m are produced and subsequently collected on a moving belt. The web is then calendered to increase its strength between a pair of heated rollers, where one roller has a raised pattern PS1. The temperature of the calender rollers (smooth roller / patterned roller) is 157°C/ 161°C and the pressure applied is about 75 N/mm. The COF used to determine the Material Factor is measured on the textured side of the web.

Sample 2 - BPB

A spunbond type nonwoven web is produced via a continuous process using three beams. The first and third beams are fed a composition that consists essentially of about 82% by weight of a polypropylene homopolymer (Tatren HT2511 from Slovnaft Petrochemicals), 16% by weight of propylene copolymere (Vistamaxx 6202 from Exxon) and about 2% by weight of a softeness enhancer additive containing 10% erucamide (CESA PPA0050079 from Clariant). The second beam is fed a composition that consists essentially of a polypropylene homopolymer (Tatren HT2511 from Slovnaft Petrochemicals). At the first and third beams, the temperature of polymeric composition measured after the extruder zone is between 245-252°C.Melt spun monocomponent filaments with a fiber diameter of between15-25 $\mu$m are produced and subsequently collected on a moving belt. The web is then calendered to increase its strength between a pair of heated rollers, where one roller has a raised pattern PS1. The temperature of the calender rollers (smooth roller / patterned roller) is 160°C/ 164°C and the pressure applied is about 75 N/mm. The COF used to determine the Material Factor is measured on the textured side of the web.

Sample 3 - PB+B+

A spunbond type nonwoven web is produced via a continuous process using three beams. The first beam is fed a composition that consists essentially of a polypropylene homopolymer (Tatren HT2511 from Slovnaft Petrochemicals). The second and third beams are fed a composition that consists essentially of about 80% by weight of a polypropylene homopolymer (Tatren HT2511 from Slovnaft Petrochemicals), about 18% by weight of propylene copolymere (Vistamaxx 6202 from Exxon) and about 2% by weight of a softness enhancer additive containing 10% erucamide (CESA PPA0050079 from Clariant). At the second and third beams the temperature of polymeric composition measured after the extruder zone is between 245-252°C. Melt spun monocomponent filaments with a fiber diameter of between15-25 $\mu$m are produced and subsequently collected on a moving belt. The web is then calendered to increase its strength between a pair of heated rollers, where one roller has a raised pattern PS1. The temperature of the calender rollers (smooth roller / patterned roller) is 160°C/ 164°C and the pressure applied is about 75 N/mm. The COF used to determine the Material Factor is measured on the textured side of the web.

Sample 4 - PVB

A spunbond type nonwoven web is produced via a continuous process using three beams. The first beam is fed a composition that consists essentially of about 98% by weight of polypropylene homopolymer (Tatren HT2511 from Slovnaft Petrochemicals) and about 2% white masterbatch (CC10084467BG from PolyOne). The second is fed a composition that consists essentially of about 82% by weight of a polypropylene homopolymer (Tatren HT2511 from Slovnaft Petrochemicals), about 16% by weight propylene copolymere (Vistamaxx 6202 from Exxon) and about 2% by weight of white masterbatch (CC10084467BG from PolyOne). The third beam is fed a composition consisting essentially of about about 79% by weight of a polypropylene homopolymer (Tatren HT2511 from Slovnaft Petrochemicals), about 16% by weight of propylene copolymere (Vistamaxx 6202 from Exxon), about 2% by weight white masterbatch (CC10084467BG from PolyOne) and about 3% by weight of softness enhancer additive containing 10% erucamide (CESA PPA0050079 from Clariant). At the third beam the temperature of polymeric composition measured after the extruder zone is between 245-252°C. Melt spun monocomponent filaments with a fiber diameter of between15-25 $\mu$m are produced and subse-quently collected on a moving belt. The web is then calendered to increase its strength between a pair of heated rollers, where one roller has a raised pattern PS1. The temperature of the calender rollers (smooth roller / patterned roller) is 160°C/ 164°C and the pressure applied is about 75 N/mm. The COF used to determine the Material Factor is measured on the textured side of the web.

Sample 5 - PVB

A spunbond type nonwoven web is produced via a continuous process using three beams. The first beam is fed a composition that consists essentially of a polypropylene homopolymer (Tatren HT2511 from Slovnaft Petrochemicals). The second beam is fed a composition that consists essentially of about 84% by weight of a polypropylene homopolymer (Tatren HT2511 from Slovnaft Petrochemicals), about 16% by weight of a propylene copolymere (Vistamaxx 6202 from Exxon). The third beam is fed a composition consisting essentially of about 81% by weight of a polypropylene homopolymer (Tatren HT2511 from Slovnaft Petrochemicals), about 16% by weight of a propylene copolymere (Vistamaxx 6202 from Exxon)and about 3% by weight of softness enhancer additive containing 10% erucamide (CESA PPA0050079 from Clariant). At the third beam the temperature of polymeric composition measured after the extruder zone is between 245-252°C. Melt spun monocomponent filaments with a fiber diameter of between15-25 $\mu$m are produced and subsequently collected on a moving belt. The web is then calendered to increase its strength between a pair of heated rollers, where one roller has a raised pattern PI. The temperature of the calender rollers (smooth roller / patterned roller) is 160°C/ 164°C and the pressure applied is about 75 N/mm. The COF used to determine the Material Factor is measured on the textured side of the web.

Sample 6 - PBB

A spunbond type nonwoven web is produced via a continuous process using three beams. The first beam is fed a composition that consists essentially of a polypropylene homopolymer (Tatren HT2511 from Slovnaft Petrochemicals). The second and third beams are fed a composition that consists essentially of about 82% by weight of a polypropylene homopolymer (Tatren HT2511 from Slovnaft Petrochemicals), about 16% by weight of a propylene copolymere (Vistamaxx 6202 from Exxon) and about 2% by weight of a softness enhancer additive containing 10% erucamide (CESA PPA0050079 from Clariant). At the second and third beams the temperature of polymeric composition measured after the extruder zone is between 245-252°C.Melt spun monocomponent filaments with a fiber diameter of between15-25 $\mu$m are produced and subsequently collected on a moving belt. The web is then calendered to increase its strength between a pair of heated rollers, where one roller has a raised pattern PS2. The temperature of the calender rollers (smooth roller / patterned roller) is 160°C/ 164°C and the pressure applied is about 75 N/mm. The COF used to determine the Material Factor is measured on the textured side of the web.

Sample 7 - PPB

A spunbond type nonwoven web is produced via a continuous process using three beams. The first and second beams are fed a composition that consists essentially of a polypropylene homopolymer (Tatren HT2511 from Slovnaft Petrochemicals). The third beams is fed a composition that consists essentially of about 82% by weight of a polypropylene homopolymer (Tatren HT2511 from Slovnaft Petrochemicals), about 16% by weight of a propylene copolymere (Vistamaxx 6202 from Exxon) and about 2% by weight of a softness enhancer additive containing 10% erucamide (CESA PPA0050079 from Clariant). At the third beam the temperature of polymeric composition measured after the extruder zone is between 245-252°C. Melt spun monocomponent filaments with a fiber diameter of between15-25 $\mu$m are produced and subsequently collected on a moving belt. The web is then calendered to increase its strength between a pair of heated rollers, where one roller has a raised pattern PS2. The temperature of the calender rollers (smooth roller / patterned roller) is 160°C/ 164°C and the pressure applied is about 75 N/mm. The COF used to determine the Material Factor is measured on the textured side of the web.

Sample 8 - B+PP

A spunbond type nonwoven web is produced via a continuous process using three beams. The first beam is fed a composition that consists essentially of about 79.5% by weight of a polypropylene homopolymer (Tatren HT2511 from Slovnaft Petrochemicals), about 18% by weight of a propylene copolymere (Vistamaxx 6202 from Exxon), and about 2.5% by weight of a softness enhancer additive containing 10% erucamide (CESA PPA0050079 from Clariant). The second and third beams are fed a composition that includes 100% by weight of a polypropylene homopolymer (Tatren HT2511 from Slovnaft Petrochemicals). At the first beam the temperature of polymeric composition measured after the extruder zone is between 245-252°C. Melt spun monocomponent filaments with a fiber diameter of between15-25 $\mu$m are produced and subsequently collected on a moving belt. The web is then calendered to increase its strength between a pair of heated rollers, where one roller has a raised pattern PS2. The temperature of the calender rollers (smooth roller / patterned roller) is 160°C/ 164°C and the pressure applied is about 75 N/mm. The COF used to determine the Material Factor is measured on the substantially flat side of the web.

S ample 9 - PPP

A spunbond type nonwoven web is produced via a continuous process using three beams. Each of the beams is fed a polymeric composition that consists essentially of a polypropylene homopolymer (Tatren HT2511 from Slovnaft Petrochemicals). Melt spun monocomponent filaments with a fiber diameter of between15-25 $\mu$m are produced and subsequently collected on a moving belt. The web is then calendered to increase its strength between a pair of heated rollers, where one roller has a raised pattern PS1. The temperature of the calender rollers (smooth roller / patterned roller) is 165°C/ 168°C and the pressure applied is about 75 N/mm. The COF used to determine the Material Factor is measured on the textured side of the web.

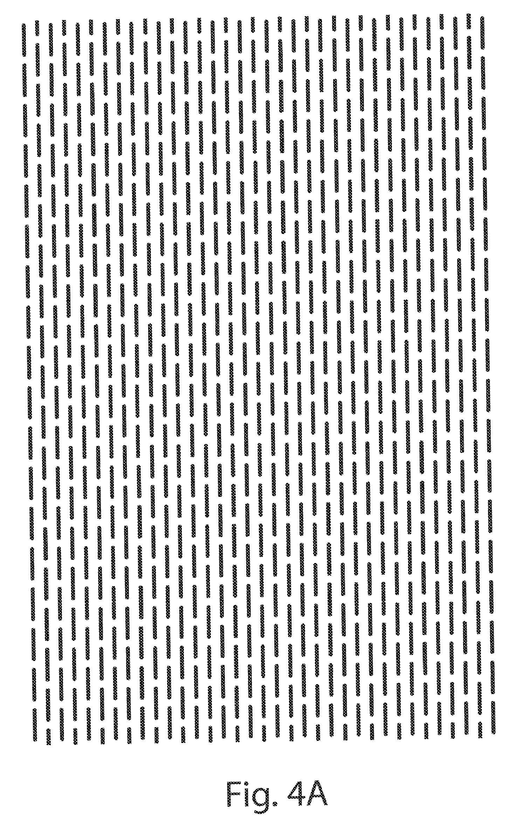

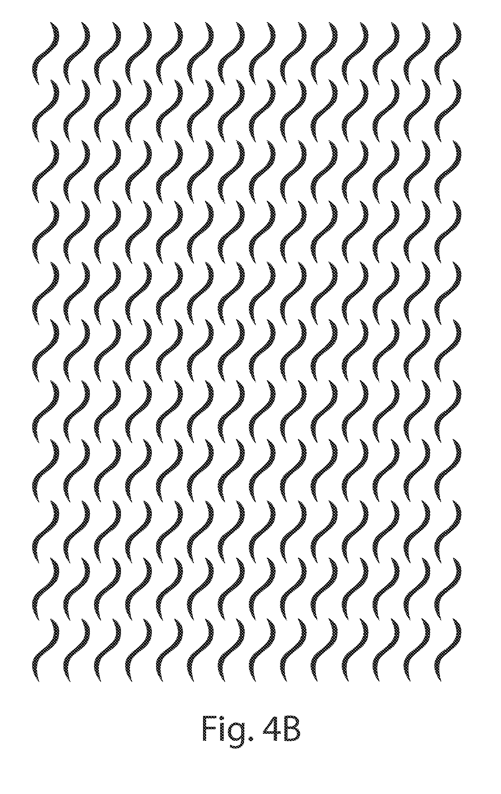

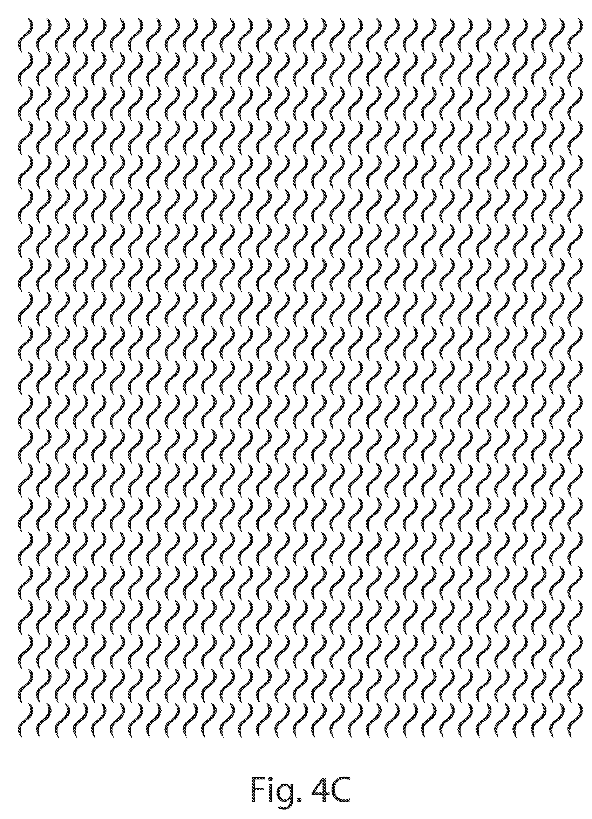

The table below summarizes the characteristics such as the % bond area and the number of bonds per cm$^2$ of the three bond patterns that are used to make samples 1-9. The bond pattern identified as PI is schematically represented in Figure 4A, the bond pattern indentified as PS1 is schematically represented in Figure 4B and the bond pattern indentified as PS2 is schematically represented in Figure 4C.

| Bond Pattern | PI | PS1 | PS2 |
|---|---|---|---|
| % bond area | 14% | 13% | 13% |
| Number of Protrusions / cm$^2$ | 9.0 | 1.5 | 2.4 |
| Protrusion greatest measurable length L in mm | 3.4 | 12.2 | 9.2 |
| Protrusion greatest measurable width W in mm | 0.4 | 4.0 | 3.0 |
| * greatest measurable length L and width W are measured as disclosed in U.S. patent application having Serial No. 13/428,404 | | | |

It should be noted that even though the nonwoven web sample 1 has good softness properties, it is prone to necking as demonstrated by a low Neckdown Modulus value. Conversely, the nonwoven web of sample 9 is not prone to necking as demonstrated by its high Neckdown Moduls but this material has rather poor softness characteristics. The Material Factor for both nonwoven webs of sample 1 and sample 9 is below 2. It is believed that nonwoven webs having a Material Factor greater than 2 such as the materials obtained for sample 2 through sample 8 offer the advantageous combination of having good softness properties, while maintaining good mechanical properties such as a relatively higher Neckdown Modulus. Some of samples provide various benefits that be may suitable for specific applications. For example, the nonwoven web of sample 2 (BPB) can be advantageously used in applications which require both outer facing surfaces of the nonwoven web to have good softness/tactile properties. A non-limiting example of such an application is the use of such a material to manufacture the front ears of a diaper. It is common for a caregiver to grab the front ears of a diaper between her index and her thumb thereby touching both sides of the front ear when she applies the diaper on a baby. Another non-limiting example of such an application is the use of such a nonwoven web to make a wipe that is used to clean a person's skin whether the wipe is a facial, body or baby wipe.

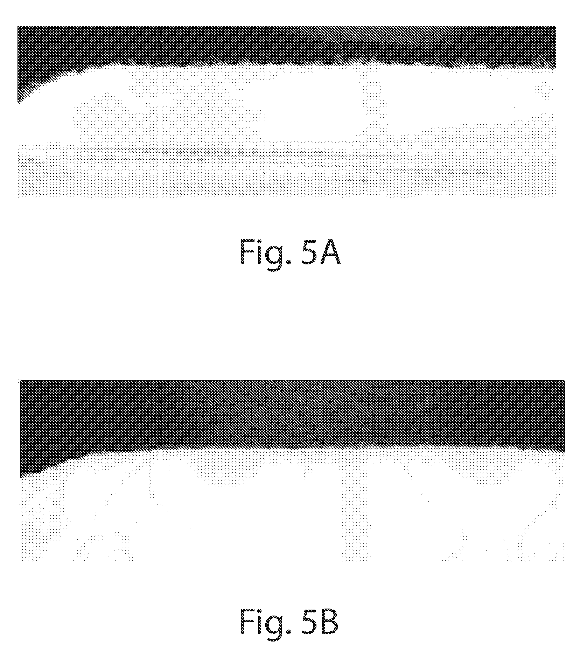

Sample 7 (PPB) and sample 8 (B+PP) are essentially mirror images of each other from a layer arrangement point of view. It should be noted however that the B layer in sample 7 is the layer that contacts the embossed roll during calendering whereas the B+ layer of sample 8 contacts the smooth roll during calendering. Both nonwovens of sample 7 and 8 may find suitable use as the outer cover or as the topsheet of an absorbent article as long as the web is disposed on the article such that the B layer of these webs is the layer that contacts the consumer or wearer's skin in use. As previously discussed, the P layers of these webs improves the mechanical properties of the webs. In addition, the P layer is better suited for adhesive or mechanical or thermo bonding of the web to other substrates to form for example a liquid impervious film to form a backsheet. It is believed that the presence of a prolypropylene homopolymer in these layers strengthens the mechanical or thermo bonds to layer(s) that also include a polypropylene. It is believed that sample 8 (BPP) may be particularly well suited in applications where "free" standing fibers extending from the surface of the material may be perceived as a negative. Figure 5A is an enlarged picture of a diaper that has been folded and placed against a dark background. The diaper includes an outercover made of the nonwoven web of sample 7 such that the B layer is the outermost layer (i.e. the layer directly facing the garment and away from the baby skin). Several "free" fibers can be seen that extend from the web. Figure 5B is an enlarged picture of a similar diaper that has been folded and also been placed against a dark background. The diaper of Figure 5B includes an outercover made of the nonwoven web of sample 8 such that the B+ layer is the outermost layer (i.e. the layer directly facing the garment and away from the baby skin). We can observe that there are significantly less "free" fibers that extend from the surface of the outercover.

In applications requiring even more softness, a nonwoven made similar to sample 6 (PBB) can be used to further increase the thickness of the soft layer.

For soft and highly extensible applications, the nonwoven webs of samples 4 and 5 (PXB compositions, where X contain certain amount of elastomer) may be preferred.

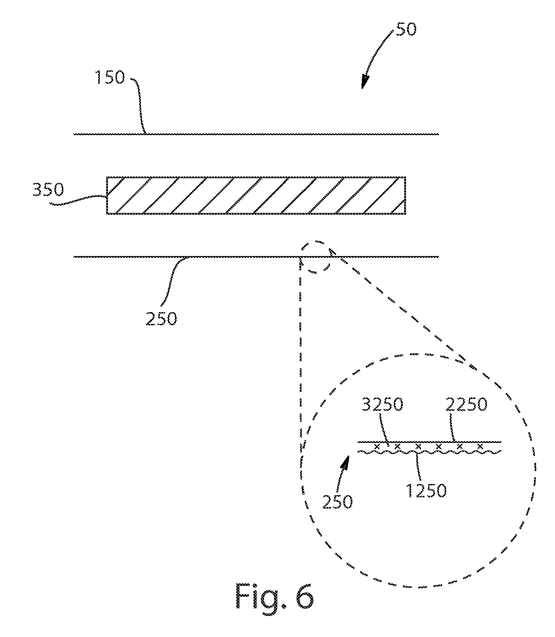

Figure 6 shows a schematic cross-section view of a product, more particularly, an absorbent article 50 that may benefit from the use of any of the nonwoven webs 10 previously discussed. The disposable absorbent article includes a liquid pervious web 150, a liquid impervious web 250 and an absorbent core 350 disposed between the liquid pervious and liquid impervious webs as is well known in the art. In one embodiment, the liquid pervious web 150 comprises a nonwoven web that includes at least a top fibrous layer and a bottom fibrous layer. The top fibrous layer comprises fibers, preferably spunbond fibers that are made of a first composition comprising a first polyolefin, a second polyolefin, which is a propylene copolymer, and a softness enhancer additive. The bottom fibrous layer comprises fibers, preferably spunbond fibers that are made of a second composition that comprises less than 10%, or less than 8%, or less than 5%, or even less than 1% by weight of the second composition of a propylene copolymer. In one embodiment, it may be advantageous for second composition to only contain an insignificant amount of, or no propylene copolymer in order to form a second fibrous layer that is less prone to necking than a first fibrous layer in particular when the second fibrous layer is subjected to a force oriented substantially in the machine direction of the second fibrous layer. A second composition may contain at least 80%, or at least 90%, or even at least 97% by weight of the second composition of a polypropylene homopolymer. The nonwoven web may also include intermediate layers as previously discussed. The resulting nonwoven web may have a basis weight of between 5 g/m$^2$ and 150 g/m$^2$, or basis weight of between 5 g/m$^2$ and 75 g/m or even between basis weight of between 5 g/m$^2$ and 30 g/m$^2$. In one embodiment, the first composition comprises at least 70%, or at least 75%, or even at least 80% by weight of the first composition of a first polyolefin, between 14% and 20%, or between 15% and 19%, or even between 16% and 18% by weight of the first composition of a second polyolefin and between 0.5% and 5%, or between 1% and 3%, or even between 1.5% and 2.5% by weight of the first composition of a softness enhancer additive masterbatch, which includes 10% by weight of active substance. The first polyolefin can advantageously be a polypropylene homopolymer. The second polyolefin can advantageously be a propylene copolymer as described *supra.* The softness enhancer additive agent can advantageously have a melting point between 75 to 112 °C, or even from 75 to 82 °C such as with Erucamide. As previously discussed, the nonwoven web can be subjected to a calendering process to provide the nonwoven web with a plurality of calendar bonds forming a pattern of bond sites on the nonwoven web. The calendering of the nonwoven web also causes one of the fibrous layers to have a three-dimensional texture as shown in Figure 1 and 2. It can therefore be advantageous for the nonwoven web to be subjected to the calendering process such that the top fibrous layer comes in direct contact with the embossing roll and the bottom fibrous layer is in direct contact with the smooth roll. A liquid pervious layer 150 that comprises such a nonwoven web can be present in the absorbent article such that the bottom fibrous layer is disposed between the top fibrous layer and the absorbent core 350 of the article. In this configuration, one of ordinary skill will understand that the top fibrous layer may be in direct contact with a person, and in particular a wearer's skin during use and provide the intended softness benefits to the liquid pervious layer. When any of the previously discussed nonwoven webs are used as part of a liquid pervious web of an absorbent article, it can be beneficial to add a surfactant to the nonwoven web in order to render the nonwoven web hydrophilic. In one embodiment, a liquid pervious layer 150 may comprise a nonwoven web in any of the configurations previously discussed that comprises at least a first layer of fibers that are made of a first composition comprising a propylene copolymer and least a second layer of fibers that are made of a second composition comprising a propylene copolymer, wherein the amount of said propylene copolymer by weight of said second composition is different than the amount of said propylene copolymer by weight of said first composition, and wherein said nonwoven web has a Material Factor of at least 2, or at least 2.5, or at least 3, or even at least 4.

In one embodiment the liquid impervious web 250 comprises a nonwoven web 1250 that is joined to a liquid impervious layer 2250, which is preferably a film, by an adhesive 3250. The nonwoven web 1250 includes at least a top fibrous layer and a bottom fibrous layer. The bottom fibrous layer comprises fibers, preferably spunbond fibers that are made of a first composition comprising a first polyolefin, a second polyolefin, which is a propylene copolymer, and a softness enhancer additive. The top fibrous layer comprises fibers, preferably spunbond fibers, that are made of a second composition that comprises less than 10%, or less than 8%, or less than 5%, or even less than 1% by weight of the second composition of a propylene copolymer. In one embodiment, it may be advantageous for second composition to only contain an insignificant amount of, or no propylene copolymer in order to form a second fibrous layer that is less prone to necking than a first fibrous layer in particular when the second fibrous layer is subjected to a force oriented substantially in the machine direction of the second fibrous layer. A second composition may contain at least 80%, or at least 90%, or even at least 97% by weight of the second composition of a polypropylene homopolymer. The nonwoven web may also include intermediate layers as previously discussed. The resulting nonwoven web may have a basis weight of basis weight of between 5 g/m$^2$ and 150 g/m$^2$, or basis weight of between 5 g/m$^2$ and 75 g/m or even between basis weight of between 5 g/m$^2$ and 30 g/m$^2$. In one embodiment, the first composition comprises greater than 75%, preferably greater than 80% by weight of a first polyolefin, between 14% and 20%, or preferably between 15% and 18%, by weight of a second polyolefin and between 0.5% and 5%, or between 1% and 3%, or even between 1.5% and 3% by weight of a softness enhancer masterbatch with 10% content of active substance. The first polyolefin can advantageously be a polypropylene homopolymer. The second polyolefin can advantageously be a propylene copolymer as described below. The softness enhancer additive can advantageously have melting point between 75 to 112 °C, preferable from 75 to 82 °C such as with Erucamide. As previously discussed, the nonwoven web can be subjected to a calendering process to provide the nonwoven web 1250 with a plurality of calendar bonds forming a pattern of bond sites on the nonwoven web. The calendering of the nonwoven web also causes one of the fibrous layers to have a three-dimensional texture as shown in Figure 1 and 2. It can therefore be advantageous for the nonwoven web to be subjected to the calendering process such that the top fibrous layer comes in direct contact with the embossing roll and the bottom fibrous layer is in direct contact with the smooth roll. A nonwoven 1250 that forms part of the liquid impervious web 250 can be present in the absorbent article such that the top fibrous layer of the nonwoven web 1250 is disposed between the bottom fibrous layer of the nonwoven web 1250 and the absorbent core 350, and in particular between the bottom fibrous layer of the nonwoven web 1250 the liquid impervious layer 2250 of the article. Without intending to be bound by any theory, it is believed that a nonwoven web having a top layer comprising less than 10% by weight of propylene copolymer can be joined more effectively to another layer such as a polymeric film with an adhesive than a nonwoven web having a top layer comprising more than 10% by weight of a propylene copolymer and/or a softness enhancer additive as previously described. In this configuration, one of ordinary skill will understand that the bottom fibrous layer may be in direct contact with a person and in particular a caregiver's skin when the caregiver is fitting the article on, for example, a baby and provide the intended softness benefits to the liquid impervious web. In one embodiment, a liquid impervious layer 250 may comprise a nonwoven web in any of the configurations previously discussed that comprises at least a first layer of fibers that are made of a first composition comprising a propylene copolymer and least a second layer of fibers that are made of a second composition comprising a propylene copolymer, wherein the amount of said propylene copolymer by weight of said second composition is different than the amount of said propylene copolymer by weight of said first composition, and wherein said nonwoven web has a Material Factor of at least 2, or at least 2.5, or at least 3, or even at least 4. It will be understood that articles that includes the previously described nonwoven web as part of the liquid pervious layer and as part of the liquid pervious web of the article are also within the scope of the invention. Any of the nonwoven webs previously described may also be incorporated into other known elements of an absorbent article that may benefit from the enhanced tactile properties of the nonwoven web. Non-limiting examples of such elements include the front and/or back ears or side panels, a nonwoven landing zone adapted to retain the hooks of a mechanical fastener disposed on the ear and/or side panels, the attachment wings of a sanitary napkin, elasticized barrier leg cuffs and waist bands disposed on the inner or outer surface of the article. Any of the previously discussed nonwoven webs may also be form part or the whole of other products such as wipes (substantially dry or pre-moistened) or article of clothing (surgical gowns, facial mask or mitts), that may benefit from the added softness of the material with reduced amount of necking. It should also be noted that the fibers that are used to make any of the individual layers ultimately forming the nonwoven web can be continuous (long) filaments (fibers) and/or discontinuous (short) filaments (fibers) obtained by processes such as, for example, spunbonding, meltblowing, carding, film fibrillation, melt-film fibrillation, airlaying, drylaying, wetlaying with staple fibers, and combinations of these processes as known in the art

Test methods:

The "basis weight" of a nonwoven web is measured according to the European standard test EN ISO 9073-1:1989 (conforms to WSP 130.1). There are 10 nonwoven web layers used for measurement, sample size 10x10 cm2. It may be advantageous for the nonwoven material to have a Basis Weight of less than 150 gsm, or less than 75 gsm, or even less than 30 gsm. The Basis Weight may also be greater than 5 gsm, or greater than 10 gsm, or even greater than 15 gsm.

The Static COF in the machine direction of the web can be measured using ASTM Method D 1894-01 with the following particulars. The test is performed on a constant rate of extension tensile tester with computer interface (a suitable instrument is the MTS Alliance using Testworks 4 Software, as available from MTS Systems Corp., Eden Prarie, MN) fitted with a coefficient of friction fixture and sled as described in D 1894-01 (a suitable fixture is the Coefficient of Friction Fixture and Sled available from Instron Corp., Canton, MA). The apparatus is configured as depicted in Figure 1c of ASTM 1894-01 using a stainless steel plane with a grind surface of 320 granulation as the target surface. A load cell is selected such that the measured forces are within 10% to 90% of the range of the cell. The tensile tester is programmed for a crosshead speed of 127 mm/min, and a total travel of 130 mm. Data is collected at a rate of 100 Hz.

To obtain the specimen from a diaper, first identify the machine direction on either the backsheet or topsheet depending on which surface is to be tested, which is typically along the longitudinal axis of the diaper. Carefully remove the nonwoven web layer from the backsheet or topsheet of sufficient size to yield a specimen. A cryogenic spray, such as CYTO-FREEZE (Control Company, Houston, TX), may be used to deactivate adhesives and enable easy separation of the nonwoven web layer from the underlying film layer. Precondition the specimens at about 23 °C $\pm$ 2 C° and about 50% $\pm$ 2% relative humidity for 2 hours prior to testing, which is performed under these same conditions. The specimen is cut to a size of 64 mm by 152 mm, with the 152 mm dimension cut parallel to the longitudinal axis of the diaper. Cut a 25mm slit in the center of one of the short ends of the specimen. Place the sled on the specimen so that the 25mm slit is aligned with the hook where the wire is connected. Pull up the slit end of the specimen so that the hook passes through the 25mm slit, and secure the ends of the strip with tape or velcro to the top of the sled. Wrap the opposite end of the specimen around the sled without slack, but without stretching, and secure that end with tape or velcro to the top of the sled. The entire bottom surface of the sled should be covered with a continuous, smooth covering of specimen. The specimen is oriented on the sled such that the wearer-facing surface, or outward- facing surface (as on the diaper, according to whether the specimen was taken from topsheet or backsheet) will face the target surface, and the longitudinal orientation of the specimen, relative the longitudinal axis of the diaper, is parallel to the pull direction of the sled. The mass of the sled with mounted specimen is recorded to 0.1 gram. The target surface of the stainless steel plane is cleaned with isopropanol before each test. In order to acquire CoF between nonwovens, a obtain a second specimen, duplicate to the one mounted to the sled, which is large enough to cover the target surface. Place the second specimen on the target surface, oriented so that the same surface of the two specimens will face each other during the test with the machine direction parallel to the pull direction of the sled. Align the specimen on the target surface so that it is equidistant between the edges. Align the end of the specimen with the protruding end of the platform, and fix it using tape or clamps along the entire protruding end only, leaving the other end of the specimen unsecured to prevent buckling of the material during testing.

The Static and Kinetic coefficients of friction (COF) for the specimen are calculated as follows:

$$\text{Static COF} = A_S / B$$

$A_S$ = maximum peak force in grams force (gf) for the initial peak
B = mass of sled in grams

$$\text{Kinetic COF} = A_K / B$$

$A_K$ = average peak force in grams force (gf) between 20 mm and 128 mm
B = mass of sled in grams

Testing is repeated for a total of 10 replicates of each specimen. Average and report the Static and Kinetic COF values for the replicates. The Static COF in the MD of the material is used to determine the Material Factor. It may be advantageous for the nonwoven material to have a Static COF in the MD of less than 0.55, or less than 0.5, or even less than 0.45. The Static COF in the MD may also be greater than 0.2, or greater than 0.25, or even greater than 0.3.

The Caliper of the nonwoven material is measured according to the European standard test EN ISO 9073-2:1995 (conforms to WSP 120.6) with following modification:

1. the material shall be measured on a sample taken from production without being exposed to higher strength forces or spending more than a day under pressure (for. example on a product roll), otherwise before measurement the material has to lie freely on a surface for at least 24 hours.
2. the overall weight of upper arm of the machine including added weight is 130 g.

It may be advantageous for the nonwoven material to have a Caliper of at least 0.1mm, or at least 0.15mm, or even at least 0.2mm. The Caliper may also be less than 2mm, or less than 1mm, or even less than 0.6mm.

The Fuzz test is performed to gravimetrically measure the amount of loose fibers collected from a nonwoven material after abrasion with sandpaper. The nonwoven can be oriented to test in either the CD and/or MD direction. The test is performed using a Model SR 550 Sutherland Rub Tester (available from Chemsultants, Fairfield OH) with the 906 g abradent weight block supplied with the instrument. A 50.8 mm wide cloth, 320 grit aluminum oxide sandpaper (available as Part No. 4687A51 from McMaster-Carr Supply Co., Elmhurst, IL) is used as the abrading surface. Fibers are collected using a 50.8 mm wide polyethylene protective tape (available as 3M Part No. 3187C). The nonwoven is mounted to the Rub tester's base plate (steel, 205 mm long x 51 mm wide x 3 mm thick) using a 50.8 mm wide double-sided tape (available as 3M Part No. 9589). All tape materials and samples are conditioned at 23 °C $\pm$ 2 C° and 50 % $\pm$ 2 % relative humidity for two hours prior to testing. All analyses are also performed in a lab maintained at 23 °C $\pm$ 2 C° and 50 % $\pm$ 2 % relative humidity.

Cut a 160 mm by 50.8 mm piece of the sandpaper. Mount the sandpaper onto the abradent weight block using its side clips. A new piece of sandpaper is used for every specimen. Cut a piece of the fiber collecting tape approximately 165 mm long by 50.8 wide. On both 50.8 wide ends, fold approximately 6 mm of the tape over onto itself (i.e., adhesive side to adhesive side) to provide a flap at each end to hold the tape without touching the adhesive. Two fiber collecting tapes are prepared for each specimen.

Place the sample to be tested flat on a lab bench with the outward facing surface, relative to the article, facing downward. Identify the CD direction of the nonwoven. Cut a piece of the sample mounting tape approximately 130 mm long by 50.8 mm wide. Place the exposed adhesive side of the tape onto the surface of the nonwoven with its longest side parallel to the CD of the nowoven. Using a paper cutter, cut a strip, 110 mm $\pm$ 1 mm in the CD direction and 40 mm $\pm$ 1 in the MD from the tape nonwoven sandwich. Remove the release paper from the specimen and adhere the specimen to the steel base plate centering the sample in the length and width dimensions. Gently place a 2.2 Kg weight block (flat-bottom, rectangular surface 50 mm wide by 150 mm long) covering the specimen for 20 sec $\pm$ 1 sec. Remove the weight.

Mount the base plate on the Sutherland Rub tester. Attach the abradent weight block onto the reciprocating arm. Start the Rub tester and allow to run for 20 cycles at a rate of 42 cycles per minute. Using an analytical balance measure the mass of each fiber collecting tape to the nearest 0.0001 g. Record separately as the sandpaper-tape tare weight (STW) and the nonwoven-tape tare weight (NTW).

After 20 cycles, carefully lift off the abradent weight block and place it on the lab bench with the sandpaper side facing upward. Take the preweighed sandpaper-fiber collecting tape and lightly touch the tape's adhesive surface to the loose fibers on the surface of the sandpaper. Care is taken to remove all loose fibers from the entire abrading surface of the sandpaper. Measure the mass of the fiber collecting tape/loose fibers to the nearest 0.0001 g. Record as the sandpaper-tape combined weight (SCW).

Carefully remove the base plate with the abraded specimen and place it on the lab bench with the nonwoven facing upward. Take the preweighed nonwoven-fiber collecting tape and cover the surface of the nonwoven with the adhesive side of the tape facing the nonwoven. Gently place a 2.2 Kg weight block (flat-bottom rectangular surface 50 mm wide by 150 mm long) covering the specimen for 20 sec $\pm$ 1 sec. Remove the weight.

Care is taken to remove all loose fibers from the entire surface of the nonwoven. Replace the release paper and measure the mass of the nonwoven-fiber collecting tape/loose fibers to the nearest 0.0001 g. Record as the non-woven-tape combined weight (NCW).

$$\text{Fuzz level (mg/cm}^2\text{)} = 1000 \text{ x } [(\text{SCW - STW}) + (\text{NCW} - \text{NTW})] / 44$$

Repeat testing on a total of three substantially identical samples Average the results and report the CD Fuzz Level to the nearest 0.001 mg/cm$^2$.

In like fashion, repeat fuzz testing for three substantially identical samples in which the specimen is oriented parallel to the MD for analysis. Average the three MD results and report the MD Fuzz Level to the nearest 0.001 mg/cm$^2$. It may be advantageous for the nonwoven material to have a Fuzz less than 0.3 mg/cm$^2$, or less than 0.25 mg/cm$^2$ or even less than 0.2 mg/cm$^2$.

The "Neckdown Modulus" can be determined as follows:

Neckdown Modulus is calculated from elongation of a specimen in the machine direction (MD) to multiple specified forces and measuring the cross direction width at the longitudinal midpoint of the specimen at each of the specified forces. The neckdown modulus is the calculated slope of the resulting force versus width curve.

All testing is performed in a conditioned room maintained at about 23 °C $\pm$ 2 C° and about 50 °C $\pm$ 2 C° relative humidity. A clean, smooth, flat, non-sticky, and unobstructed horizontal testing surface (such as a lab bench) that is at least 400 mm wide and 2 m long is required for testing. Force measurements are made using a force gauge with a capacity of 25 N (such as a Medio-Line 40025 available from Pesola AG, Baar, Switzerland) which has been calibrated with weights certified by NIST. Length measurements are made with a NIST traceable ruler that is graduated at 1 mm intervals and longer than the length to be measured. The specimens are pulled using a Plexiglass rod, 9.5 mm diameter and 230 mm long. The ends of a 350 mm long non-stretchable string are attached to each end of the Plexiglass rod. The cut specimens, are conditioned lying flat on a horizontal surface under no tension for at least 30 minutes at about 23 °C $\pm$ 2 C° and about 50 °C $\pm$ 2 C° relative humidity, prior to testing.

Lay the prepared sample flat on the testing surface. Mark a line on the specimen parallel to the CD, 25 mm from the MD end (MDE1). Mark a second line on the opposite MD edge (MDE2), parallel to the CD, 85 mm from the MDE2. Flip the specimen over so the back side of the specimen is facing upward. Mark a third line on the specimen parallel to the CD, 25 mm from MDE2.

Cut a piece of 2 in. wide duct tape 220 mm $\pm$ 1 mm long. Center the long edge of the tape with the longitudinal centerline of the specimen, and align the tape along the marked line such that 25 mm of the tape is applied to the specimen and 25 mm extends past the MDE2. Again flip the specimen over so that the back side of the specimen is facing the testing surface once again. Cut a piece of the 2 in wide duct tape approximately 250 mm long. At the MDE1, center the long edge of the tape with the longitudinal centerline of the specimen, and align the tape along the marked line such that 25 mm of the tape is applied to the specimen and 25 mm is applied to the test surface past the MDE1. Place the Plexiglass rod on top of the specimen with it centered along the longitudinal centerline of the specimen and next to the MDE2. Wrap the specimen over the rod and align the distal edge of the tap to the line marked 85 mm from the MDE2. The gage length between the interior edges of tapes is 1320 mm $\pm$ 1 mm. Mark the specimen at the intersection of the longitudinal centerline of the specimen and the middle of the gage length (660 mm $\pm$ 1 mm from either tape edge). Attach the force gauge to the non-stretchable string using a hook fixture.

Align the force gauge width longitudinal centerline of the specimen with minimal slack in the non-stretchable string and specimen. After the test is started, the specimen remains under the applied force for the duration of the experiment. First measure and record the CD width of the specimen at the marked midpoint of the gage to the nearest 0.1 mm. Manually pull the force gauge at a rate of approximately 100 mm/sec along the projected specimen centerline until the force gauge measures 2.0 N $\pm$ 0.2 N. After 30 sec, measure and record the CD width at the marked midpoint of the gage to the nearest 0.1mm. Also record the applied force to the nearest 0.01N. Repeat this measure for every incremental 2 N, with 24 N being the last measured point.

Plot the values of Applied Force (in N) versus Specimen CD Width (in m). Fit a least squares linear regression of the line and report the slope as Neckdown Modulus (N/m) to the nearest 1 N/m. Repeat the test for five substantially similar specimens and report as the average to the nearest 1 N/m.

It may be advantageous for the nonwoven material to have a Neck down modulus of at least 3,5 N/cm, or at least 4 N/cm, or at least 5,5 N/cm, or even at least 7 N/cm.

As previously discussed, the "Flexular modulus" can be determined according to the standard method ASTM D790. It is also believed that a nonwoven web that includes at least a first layer of fibers made of a first composition comprising a first polyolefin, a second polyolefin, and a softness enhancer additive, such that second polyolefin is a propylene copolymer and such that the second polyolefin is a different polyolefin than said first polyolefin is less prone to necking when the nonwoven web also includes at least a second layer of fibers that are made of a second composition and such that the Flexular Modulus of the second composition is greater than the Flexular Modulus of the first composition.

As previously discussed, any of the nonwoven webs of the invention described hereinbefore may also be advantageously used in any other products that may benefit from improved tactile properties.

## Claims

1. An article comprising a liquid pervious layer, a liquid impervious layer, an absorbent core disposed between said liquid pervious layer and said liquid impervious layer and a nonwoven web, said nonwoven web being **characterized in that** it comprises:

   at least a first layer of fibers that are made of a first composition comprising a first polyolefin, a second polyolefin, and a softness enhancer additive, wherein said second polyolefin is a propylene copolymer and wherein said second polyolefin is a different polyolefin than said first polyolefin; and at least a second layer of fibers that are made of a second composition,
   wherein the Flexular Modulus of the second composition is greater than the Flexular Modulus of the first composition.

2. The article according to claim 1 **characterized in that** said nonwoven web is present in said absorbent article such that said second layer of fibers is disposed between said first layer of fibers and said absorbent core

3. The article according to any preceding claims **characterized in that** the fibers of said first and said second layers are spunbond fibers.

4. The article according to any preceding claims **characterized in that** said nonwoven web comprises a plurality of calendering bonds that provide said nonwoven web with a first textured surface and a second surface opposite said first surface.

5. The absorbent article according to any preceding claims **characterized in that** said first composition used to make said fibers of said first layer comprises between 5% and 25% of said second polyolefin and between 0.01% to 10% of softness enhancer additive by weight of said fibers.

6. The article according to any preceding claims **characterized in that** said nonwoven web comprises at least an intermediate fibrous layer present between said first and second fibrous layers wherein said intermediate fibrous layer comprises fibers that are made of a third composition, preferably said third composition comprises less than 10% by weight of said third composition of a propylene copolymer, preferably said third composition comprises more than 10% by weight of said third composition of a propylene copolymer.

7. The article according to any preceding claims **characterized in that** each of said liquid impervious backsheet and said liquid pervious topsheet comprises a nonwoven web comprising at least a first layer of fibers that are made of a first composition comprising a first polyolefin, a second polyolefin, and a softness enhancer additive, wherein said second polyolefin is a propylene copolymer and wherein said second polyolefin is a different polyolefin than said first polyolefin and at least a second layer of fibers that are made of a second composition comprising less than 10% by weight of said second composition of a propylene copolymer and wherein said nonwoven web is present in said absorbent article such that said second layer of fibers is disposed between said first layer of fibers and said absorbent core.

8. The article according to any preceding claims **characterized in that** said liquid pervious layer comprises said nonwoven web such that said nonwoven web is disposed at a body facing surface of said article.

9. The article according to any preceding claims **characterized in that** said second composition comprises a polypropylene homopolymer in an amount greater than 80% by weight of said second composition.

**Patentansprüche**

1. Artikel, umfassend eine flüssigkeitsdurchlässige Lage, eine flüssigkeitsundurchlässige Lage, einen absorptionsfähigen Kern, der zwischen der flüssigkeitsdurchlässigen Lage und der flüssigkeitsundurchlässigen Lage angeordnet ist, und einen Vliesstoff, wobei der Vliesstoff **dadurch gekennzeichnet ist, dass** er Folgendes umfasst:

mindestens eine erste Faserlage, die aus einer ersten Zusammensetzung hergestellt ist, die ein erstes Polyolefin, ein zweites Polyolefin und ein Weichheitsverbessereradditiv umfasst, wobei das zweite Polyolefin ein Propylen-Copolymer ist, und wobei das zweite Polyolefin ein anderes Polyolefin als das erste Polyolefin ist; und mindestens eine zweite Faserlage, die aus einer zweiten Zusammensetzung hergestellt ist, wobei der Biegemodul der zweiten Zusammensetzung höher als der Biegemodul der ersten Zusammensetzung ist.

2. Artikel nach Anspruch 1, **dadurch gekennzeichnet, dass** der Vliesstoff in dem absorptionsfähigen Artikel derart vorliegt, dass die zweite Faserlage zwischen der ersten Faserlage und dem absorptionsfähigen Kern angeordnet ist.

3. Artikel nach einem der vorstehenden Ansprüche, **dadurch gekennzeichnet, dass** die Fasern der ersten und der zweiten Lage Spinnvliesfasern sind.

4. Artikel nach einem der vorstehenden Ansprüche, **dadurch gekennzeichnet, dass** der Vliesstoff eine Vielzahl von Kalandrierbindungen umfasst, wodurch der Vliesstoff mit einer ersten texturierten Oberfläche und einer zweiten Oberfläche entgegengesetzt zu der ersten Oberfläche versehen wird.

5. Absorptionsfähiger Artikel nach einem der vorstehenden Ansprüche, **dadurch gekennzeichnet, dass** die erste Zusammensetzung, die zum Herstellen der Fasern der ersten Lage verwendet wird, bezogen auf das Gewicht der Fasern zwischen 5 % und 25 % des zweiten Polyolefins und zwischen 0,01 % bis 10 % eines Weichheitsverbessereradditivs umfasst.

6. Artikel nach einem der vorstehenden Ansprüche, **dadurch gekennzeichnet, dass** der Vliesstoff mindestens eine faserige Zwischenlage umfasst, die zwischen der ersten und der zweiten faserigen Lage vorliegt, wobei die faserige Zwischenlage Fasern umfasst, die aus einer dritten Zusammensetzung hergestellt sind, wobei die dritte Zusammensetzung, bezogen auf das Gewicht der dritten Zusammensetzung, vorzugsweise weniger als 10 % eines Propylen-Copolymers umfasst, wobei die dritte Zusammensetzung, bezogen auf das Gewicht der dritten Zusammensetzung, vorzugsweise mehr als 10 % eines Propylen-Copolymers umfasst.

7. Artikel nach einem der vorstehenden Ansprüche, **dadurch gekennzeichnet, dass** jede der flüssigkeitsundurchlässigen Hinterlage und der flüssigkeitsdurchlässigen Decklage einen Vliesstoff umfasst, umfassend mindestens eine erste Faserlage, die aus einer ersten Zusammensetzung hergestellt ist, die ein erstes Polyolefin, ein zweites Polyolefin und ein Weichheitsverbessereradditiv umfasst, wobei das zweite Polyolefin ein Propylen-Copolymer ist, und wobei das zweite Polyolefin ein anderes Polyolefin als das erste Polyolefin ist, und mindestens eine zweite Faserlage, die aus einer zweiten Zusammensetzung hergestellt ist, die, bezogen auf das Gewicht der zweiten Zusammensetzung, weniger als 10 % eines Propylen-Copolymers umfasst, und wobei der Vliesstoff in dem absorptionsfähigen Artikel derart vorliegt, dass die zweite Faserlage zwischen der ersten Faserlage und dem absorptionsfähigen Kern angeordnet ist.

8. Artikel nach einem der vorstehenden Ansprüche, **dadurch gekennzeichnet, dass** die flüssigkeitsdurchlässige Lage den Vliesstoff derart umfasst, dass der Vliesstoff an einer dem Körper zugewandten Oberfläche des Artikels angeordnet ist.

**9.** Artikel nach einem der vorstehenden Ansprüche, **dadurch gekennzeichnet, dass** die zweite Zusammensetzung ein Polypropylen-Homopolymer in einer Menge von mehr als 80 %, bezogen auf das Gewicht der zweiten Zusammensetzung, umfasst.

**Revendications**

**1.** Article comprenant une couche perméable aux liquides, une couche imperméable aux liquides, une âme absorbante disposée entre ladite couche perméable aux liquides et ladite couche imperméable aux liquides et une nappe non tissée, ladite nappe non tissée étant **caractérisée en ce qu**'elle comprend :

au moins une première couche de fibres qui sont constituées d'une première composition comprenant une première polyoléfine, une deuxième polyoléfine, et un additif amplificateur de douceur, dans lequel ladite deuxième polyoléfine est un copolymère de propylène et dans lequel ladite deuxième polyoléfine est une polyoléfine différente de ladite première polyoléfine ; et
au moins une deuxième couche de fibres qui sont constituées d'une deuxième composition,
dans lequel le module de flexion de la deuxième composition est supérieur au module de flexion de la première composition.

**2.** Article selon la revendication 1, **caractérisé en ce que** ladite nappe non tissée est présente dans ledit article absorbant de telle sorte que ladite deuxième couche de fibres est disposée entre ladite première couche de fibres et ladite âme absorbante.

**3.** Article selon l'une quelconque des revendications précédentes, **caractérisé en ce que les** fibres desdites première et deuxième couches sont des fibres filées-liées.

**4.** Article selon l'une quelconque des revendications précédentes, **caractérisé en ce que** ladite nappe non tissée comprend une pluralité de liaisons par calandrage qui fournissent à ladite nappe non tissée une première surface texturée et une deuxième surface opposée à ladite première surface.

**5.** Article absorbant selon l'une quelconque des revendications précédentes, **caractérisé en ce que** ladite première composition utilisée pour fabriquer lesdites fibres de ladite première couche comprend entre 5 % et 25 % de ladite deuxième polyoléfine et entre 0,01 % et 10 % de l'additif amplificateur de douceur, en poids desdites fibres.

**6.** Article selon l'une quelconque des revendications précédentes, **caractérisé en ce que** ladite nappe non tissée comprend au moins une couche fibreuse intermédiaire présente entre lesdites première et deuxième couches fibreuses, dans lequel ladite couche fibreuse intermédiaire comprend des fibres qui sont constituées d'une troisième composition, de préférence ladite troisième composition comprend moins de 10 % en poids de ladite troisième composition d'un copolymère de propylène, de préférence ladite troisième composition comprend plus de 10 % en poids de ladite troisième composition d'un copolymère de propylène.

**7.** Article selon l'une quelconque des revendications précédentes, **caractérisé en ce que** chacune parmi ladite feuille de fond imperméable aux liquides et ladite feuille de dessus perméable aux liquides comprend une nappe non tissée comprenant au moins une première couche de fibres qui sont constituées d'une première composition comprenant une première polyoléfine, une deuxième polyoléfine et un additif amplificateur de douceur, dans lequel ladite deuxième polyoléfine est un copolymère de propylène et dans lequel ladite deuxième polyoléfine est une polyoléfine différente de ladite première polyoléfine et au moins une deuxième couche de fibres qui sont constituées d'une deuxième composition comprenant moins de 10 % en poids de ladite deuxième composition d'un copolymère de propylène et dans lequel ladite nappe non tissée est présente dans ledit article absorbant de telle sorte que ladite deuxième couche de fibres est disposée entre ladite première couche de fibres et ladite âme absorbante.

**8.** Article selon l'une quelconque des revendications précédentes, **caractérisé en ce que** ladite couche perméable aux liquides comprend ladite nappe non tissée de telle sorte que ladite nappe non tissée est disposée au niveau d'une surface faisant face au corps dudit article.

**9.** Article selon l'une quelconque des revendications précédentes, **caractérisé en ce que** ladite deuxième composition comprend un homopolymère de polypropylène en une quantité supérieure à 80 % en poids de ladite deuxième composition.

Fig. 1

Fig. 2

Fig. 3

EP 2 916 788 B1

Fig. 4A

Fig. 4B

Fig. 4C

EP 2 916 788 B1

Fig. 5A

Fig. 5B

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007140163 A
- EP 1275763 A
- US 20040005457 A
- US 3849241 A, Buntin
- US 3338992 A
- US 3692613 A
- US 3802817 A
- US 4405297 A
- US 5665300 A
- US 13428404 B, Xu
- US 428404 A

**Non-patent literature cited in the description**

- **H. N. CHENG.** *MACROMOLECULES,* 1984, vol. 17, 1950
- **VERSTATE et al.** *MACROMOLECULES,* 1988, vol. 21, 3360
- **ERNEST W.FLICK.** *Plastics additives: an industrial guide*